# EUROPEAN PATENT APPLICATION

(11) **EP 3 620 527 A1**
(43) Date of publication of application: **11.03.2020**
(21) Application number: 18794051.5
(22) Date of filing: 19.04.2018
(51) Int. Cl.: C12P 21/02, C12N 9/12

(54) **PRODUCTION METHOD FOR SUBSTANCE USING ATP**

(30) Priority: 01.05.2017 JP 2017091451
(71) Applicant: Kaneka Corporation, Osaka-shi, Osaka 530-8288 (JP)
(72) Inventor: MATSUI, Misato, Takasago-shi Hyogo 676-8688 (JP); ITO, Noriyuki, Takasago-shi Hyogo 676-8688 (JP); YASOHARA, Yoshihiko, Takasago-shi Hyogo 676-8688 (JP)
(74) Representative: Hoffmann Eitle
(86) International application number: PCT/JP2018/016175
(87) International publication number: WO 2018/203482

(57) **Abstract**

The present invention provides a novel method of producing a substance using ATP. The method is a method of producing a substance using ATP, wherein: ADP is generated from ATP during the method; the method is coupled with an ATP regeneration reaction in which a polyphosphate kinase 2 and a polyphosphoric acid are allowed to react with the ADP to regenerate ATP; and the ATP used in the method includes the ATP regenerated by the ATP regeneration reaction, the method including using, as a substrate for the polyphosphate kinase 2, a polyphosphoric acid mixture that contains polyphosphoric acid molecules with a degree of polymerization of not less than 15 in an amount of not less than 48%.

## Description

### Technical Field

The present invention relates to a novel method of producing a substance using adenosine triphosphate (ATP).

### Background Art

Glutathione is a peptide composed of the following three amino acids: L-cysteine, L-glutamic acid, and glycine. Glutathione can be found not only in human bodies but also in many other living bodies such as other animals, plants, and microorganisms. Furthermore, glutathione has the functions of eliminating reactive oxygen, detoxification, amino acid metabolism, and the like, and is a compound important to living bodies.

Glutathione *in vivo* is in the form of (i) reduced glutathione (hereinafter may be referred to as "GSH"), in which the thiol group of L-cysteine residue is in a reduced form "-SH" or (ii) oxidized glutathione (hereinafter may be referred to as "GSSG"), in which the thiol groups of L-cysteine residues of two glutathione molecules are oxidized to form a disulfide bond between the two glutathione molecules.

Examples of a known method of producing glutathione include an enzymatic production in which bodies of *Escherichia coli* and/or *Saccharomyces cerevisiae,* which have been recombined to produce γ-glutamylcysteine synthase and/or glutathione synthase, are used as enzyme sources in the presence of L-glutamic acid, L-cysteine, glycine, a surfactant, an organic solvent and/or the like (Patent Literatures 1 and 2). Furthermore, the applicant has recently disclosed a method of producing oxidized glutathione, the method including the steps of: producing oxidized γ-glutamylcysteine from L-glutamic acid and L-cystine; and then producing oxidized glutathione from the oxidized γ-glutamylcysteine and glycine (Patent Literature 3).

Examples of a known enzyme involved in glutathione synthesis include: γ-glutamylcysteine synthase (hereinafter may be referred to as "GSHI") which combines L-glutamic acid and L-cysteine to form γ-glutamylcysteine; and glutathione synthase (hereinafter may be referred to as "GSHII") which combines γ-glutamylcysteine and glycine to form reduced glutathione. The GSHI and GSHII are known to be capable of also using L-cystine and oxidized γ-glutamylcysteine as substrates, respectively. In a case where the GSHI and GSHII use L-cystine and oxidized γ-glutamylcysteine as substrates, respectively, their enzymatic reactions result in synthesis of oxidized γ-glutamylcysteine and oxidized glutathione, respectively, as reaction products (Patent Literature 3). Furthermore, bifunctional glutathione synthase (hereinafter may be referred to as "GSHF") which has both functions of the GSHI and GSHII is also known (Patent Literature 3).

### Citation List

### [Patent Literature]

[Patent Literature 1]
   Japanese Patent Application Publication, *Tokukaisho,* No. 60-27396
[Patent Literature 2]
   Japanese Patent Application Publication, *Tokukaisho,* No. 60-27397
[Patent Literature 3]
   PCT International Publication No. WO 2016/002884

### Summary of Invention

### Technical Problem

Incidentally, the GSHI, GSHII, GSHF, and the like consume adenosine triphosphate (hereinafter may be referred to as "ATP") as an energy source for their activity. Therefore, in order to maintain the reaction of glutathione production, it is necessary to externally supply ATP or it is necessary to reconvert adenosine diphosphate (hereinafter may be referred to as "ADP"), which is a product of the consumption of ATP, into ATP.

External supply of ATP is very costly; therefore, an ATP-regenerating system, in which ADP is reconverted into ATP, has been considered for application.

A known enzyme that converts ADP to ATP in the ATP-regenerating system is a polyphosphate kinase 2. This enzyme has the function of converting ADP into ATP using metaphosphoric acid or the like as a substrate.

However, a production method in which the ATP-regenerating system is included as part of the production of a substance, for example, a method of producing oxidized glutathione, has been required to have an improved ATP-regenerating system in order to achieve a higher rate of conversion from a source material into a final product (e.g., oxidized glutathione).

In view of such circumstances, it is an object of the present invention to provide a novel method of producing a substance using ATP.

### Solution to Problem

The inventors studied hard in order to attain the above object, and for the first time found that, by using, as a substrate for a polyphosphate kinase 2, a mixture that contains polyphosphoric acid molecules with a high degree of polymerization, it is possible to achieve a high rate of conversion to oxidized glutathione. On the basis of this finding, the inventors accomplished the present invention.

Specifically, an aspect of the present invention relates to a method of producing a substance using ATP, wherein: ADP is generated from ATP during the method; the method is coupled with an ATP regeneration reaction in which a polyphosphate kinase 2 and polyphosphoric acid are allowed to react with the ADP to regenerate ATP; and the ATP used in the method includes the ATP regenerated by the ATP regeneration reaction, the method including using, as a substrate for the polyphosphate kinase 2, a polyphosphoric acid mixture that contains polyphosphoric acid molecules with a degree of polymerization of not less than 15 in an amount of not less than 48%.

### Advantageous Effects of Invention

According to an aspect of the present invention, it is possible to produce a substance using ATP with a high conversion rate at low cost.

### Brief Description of Drawings

Fig. 1 is a chart showing the results of analysis of a polyphosphoric acid mixture in terms of the degree of polymerization.
Fig. 2 is a chart that shows a comparison, in terms of changes in degree of polymerization, between polyphosphoric acid mixtures which had been left to stand for different periods of time after their preparations.
Fig. 3 show charts showing how consumption of a polyphosphoric acid mixture changes during production of oxidized glutathione.

### Description of Embodiments

The following description will discuss, in detail, an embodiment of the present invention. Note that all academic and patent literatures listed herein are incorporated herein by reference.

In this specification, the term "gene" is used interchangeably with the term "polynucleotide", "nucleic acid" or "nucleic acid molecule", and is intended to mean a polymer of nucleotides. A gene can exist in the form of DNA (e.g., cDNA or genomic DNA) or RNA (e.g., mRNA). DNA or RNA may be double-stranded or single stranded. Single-stranded DNA or RNA may be a coding strand (sense strand) or may be a noncoding strand (antisense strand). A gene may be chemically synthesized, and may have codon usage modified so that the expression of a protein that the gene codes for improves. Codons which code for the same amino acid may be replaced with each other.

The term "protein" is used interchangeably with the term "peptide" or "polypeptide". In this specification, bases and amino acids are indicated by single letter codes or three letter codes of IUPAC standards and IUB standards.

### [Method of producing substance]

An embodiment of the present invention provides a method of producing a substance using ATP, wherein: ADP is generated from ATP during the method; the method is coupled with an ATP regeneration reaction in which a polyphosphate kinase 2 and polyphosphoric acid are allowed to react with the ADP to regenerate ATP; and the ATP used in the method includes the ATP regenerated by the ATP regeneration reaction, the method including using, as a substrate for the polyphosphate kinase 2, a polyphosphoric acid mixture that contains polyphosphoric acid molecules with a degree of polymerization of not less than 15 in an amount of not less than 48%.

An embodiment of the present invention was accomplished based on the following finding. The inventors for the first time found that, by arranging a method of producing a substance using ATP such that ATP is regenerated using, as a substrate for a polyphosphate kinase 2, a polyphosphoric acid mixture containing a certain amount or more of polyphosphoric acid molecules with a specific degree of polymerization (particularly, polyphosphoric acid molecules with a high degree of polymerization), it is possible to produce a substance using the ATP with a high conversion rate. As such, an embodiment of the present invention uses a polyphosphoric acid mixture that contains a certain amount or more of polyphosphoric acid molecules with a high degree of polymerization in the ATP regeneration reaction, and thereby makes it possible to produce a substance with a high conversion rate at low cost.

The following description will discuss features of the present invention in detail.

### <1. Polyphosphoric acid mixture>

In an embodiment of the present invention, it is preferable that the following are used: a polyphosphate kinase 2; and a polyphosphoric acid mixture that serves as a substrate for the polyphosphate kinase 2 and that contains polyphosphoric acid molecules with a degree of polymerization of not less than 15 in an amount of not less than 48%. In an embodiment of the present invention, use of such a polyphosphoric acid mixture that contains a certain amount or more of polyphosphoric acid molecules with a high degree of polymerization makes it possible to produce a substance with a high conversion rate at low cost.

In this specification, the term "polyphosphoric acid" is intended to mean a polymer obtained by polymerization of phosphoric acid units. For example, a polyphosphoric acid is a compound represented by Formula 1 below. 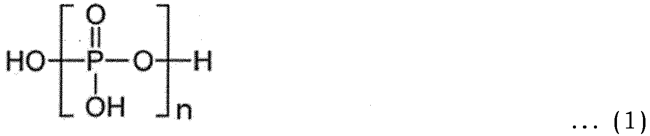 In this specification, the term "metaphosphoric acid" is intended to mean a compound that contains (i) a chain polymer structure composed of phosphoric acid units and (ii) a ring structure. A "metaphosphoric acid" is, for example, a compound that contains a compound represented by Formula (1) (corresponding to "chain polymer structure composed of phosphoric acid units") and a compound represented by Formula 2 below (corresponding to "ring structure"). 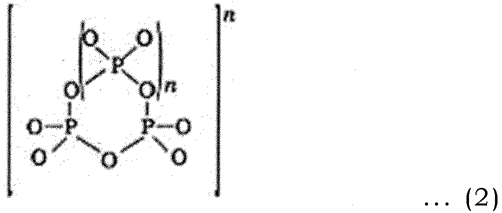 In this specification, the term "polyphosphoric acid mixture" is intended to mean a mixture that contains one of the "polyphosphoric acid" and "metaphosphoric acid" or that contains both of the "polyphosphoric acid" and "metaphosphoric acid". The proportion of the "polyphosphoric acid" and/or "metaphosphoric acid" in the "polyphosphoric acid mixture" is not particularly limited, provided that the effects of the present invention are achieved.

Note that, because the "polyphosphoric acid" and "metaphosphoric acid" can be present in a mixed manner, it is difficult to strictly separate them from each other. Therefore, in this specification, the terms "polyphosphoric acid" and "metaphosphoric acid" are not distinguished precisely. The term "polyphosphoric acid" herein means "polyphosphoric acid" that contains "metaphosphoric acid", and the term "metaphosphoric acid" herein means "metaphosphoric acid" that contains "polyphosphoric acid".

In an embodiment of the present invention, the polyphosphoric acid mixture contains polyphosphoric acid molecules with a degree of polymerization of not less than 15 in an amount of not less than 48%, preferably contains polyphosphoric acid molecules with a degree of polymerization of not less than 15 in an amount of not less than 50%.

In another embodiment of the present invention, the polyphosphoric acid mixture contains polyphosphoric acid molecules with a degree of polymerization of not less than 20 in an amount of not less than 31%, preferably contains polyphosphoric acid molecules with a degree of polymerization of not less than 20 in an amount of not less than 32%.

In a further embodiment of the present invention, the polyphosphoric acid mixture contains polyphosphoric acid molecules with a degree of polymerization of not less than 36 in an amount of not less than 4%, preferably contains polyphosphoric acid molecules with a degree of polymerization of not less than 36 in an amount of not less than 5%.

In still a further embodiment of the present invention, the polyphosphoric acid mixture contains polyphosphoric acid molecules with a degree of polymerization of not less than 43 in an amount of not less than 2%. In a still yet further embodiment of the present invention, the polyphosphoric acid mixture contains polyphosphoric acid molecules with a degree of polymerization of not less than 50 in an amount of not less than 2%.

The degree of polymerization of polyphosphoric acid molecules in an embodiment of the present invention is determined by a method that will be described later in Examples. Furthermore, examples of such a polyphosphoric acid mixture that contains a certain amount or more of polyphosphoric acid molecules with a high degree of polymerization will be provided later in Examples (see Examples 1, 4, and the like).

### <2. Polyphosphate kinase 2 (PPK2)>

An embodiment of the present invention provides a method of producing a substance, in which the polyphosphate kinase 2 is at least one selected from the group consisting of: polyphosphate kinase 2 derived from *Pseudomonas aeruginosa* (hereinafter may be referred to as "PNDK"); polyphosphate kinase 2 derived from Synechococcus sp. PCC6312 (hereinafter may be referred to as "Sy PPK2"; polyphosphate kinase 2 derived from *Corynebacterium efficiens* (hereinafter may be referred to as "CE PPK2"); polyphosphate kinase 2 derived from *Kineococcus radiotolerans* (hereinafter may be referred to as "KR PPK2"); polyphosphate kinase 2 derived from *Pannonibacter indicus* (hereinafter may be referred to as "PI PPK2"); polyphosphate kinase 2 derived from *Deinococcus radiodurans* K1 (hereinafter may be referred to as "DR PPK2"); polyphosphate kinase 2 derived from *Gulbenkiania indica* (hereinafter may be referred to as "GI PPK2"); polyphosphate kinase 2 derived from *Arthrobactor aurescens* TC1 (hereinafter may be referred to as "AA PPK2"); polyphosphate kinase 2 derived from *Thiobacillus denitrificans* ATCC25259 (hereinafter may be referred to as TD PPK2"); and polyphosphate kinase 2 derived from *Pseudomonas fluorescens* (hereinafter may be referred to as "PF PPK2").

Polyphosphate kinases (hereinafter may be referred to as "PPK") are classified into two types of enzyme for a reversible reaction: polyphosphate kinase 1 (hereinafter may be referred to as "PPK1"): and polyphosphate kinase 2 (hereinafter may be referred to as "PPK2"). It is known that the PPK1s are predominantly involved in a reaction that degrades ATP into ADP and polyphosphoric acid (hereinafter may be referred to as "PolyP") and that the PPK2s are predominantly involved in a reaction that combines ADP and PolyP to form ATP.

The PPK2s are further classified into three classes in terms of the reactions they catalyze. Class I PPK2 catalyzes a reaction that combines ADP and PolyP to form ATP, and examples thereof include PNDK. Class II PPK2 catalyzes a reaction that combines adenosine monophosphate (hereinafter may be referred to as "AMP") and PolyP to form ATP, and examples thereof include polyphosphoric-acid-dependent AMP transferase (PAP). Class III PPK2 is a bifunctional enzyme that catalyzes the two reactions of the above Class I and Class II, and examples thereof include PPK2 derived from *Meiothermus ruber.*

The inventors have studied hard in order to search for a novel PPK2, and succeeded in identifying eight types of novel PPK2 which are classified into Class I or Class III and which have the activity of combining ADP and PolyP to thereby convert ADP into ATP. This makes it possible to catalyze the ATP regeneration reaction by use of a PPK2 selected appropriately from not only conventionally-known PPK2s and DR PPK2 but also these eight types of PPK2.

Needless to say, in an embodiment of the present invention, a conventionally-known PPK2 can be employed as the polyphosphate kinase 2.

The following description discusses the above PPK2s (i.e., PNDK, DR PPK2, and eight types of novel PPK2) in detail.
The PNDK is polyphosphate kinase 2 derived from *Pseudomonas aeruginosa,* and is composed of a total of 357 amino acid residues (SEQ ID NO:1).
The Sy PPK2 is polyphosphate kinase 2 derived from Synechococcus sp. PCC6312, and is composed of a total of 296 amino acid residues (SEQ ID NO:2).
The CE PPK2 is polyphosphate kinase 2 derived from *Corynebacterium efficiens,* and is composed of a total of 351 amino acid residues (SEQ ID NO:3).
The KR PPK2 is polyphosphate kinase 2 derived from *Kineococcus radiotolerans,* and is composed of a total of 296 amino acid residues (SEQ ID NO:4).
The PI PPK2 is polyphosphate kinase 2 derived from *Pannonibacter indicus,* and is composed of a total of 367 amino acid residues (SEQ ID NO:5).
The DR PPK2 is polyphosphate kinase 2 derived from *Deinococcus radiodurans* K1, and is composed of a total of 266 amino acid residues (SEQ ID NO:6).
The GI PPK2 is polyphosphate kinase 2 derived from *Gulbenkiania indica,* and is composed of a total of 350 amino acid residues (SEQ ID NO:7).
The AA PPK2 is polyphosphate kinase 2 derived from *Arthrobactor aurescens* TC1, and is composed of a total of 314 amino acid residues (SEQ ID NO:8).
The TD PPK2 is polyphosphate kinase 2 derived from *Thiobacillus denitrificans* ATCC25259, and is composed of a total of 269 amino acid residues (SEQ ID NO:9).
The PF PPK2 is polyphosphate kinase 2 derived from *Pseudomonas fluorescens,* and is composed of a total of 362 amino acid residues (SEQ ID NO:10).

The following are base sequences which code for the above ten types of PPK2 and which are codon-optimized for expression in *E. coli:* PNDK (SEQ ID NO:11); Sy PPK2 (SEQ ID NO:12); CE PPK2 (SEQ ID NO:13); KR PPK2 (SEQ ID NO:14); PI PPK2 (SEQ ID NO:15); DR PPK2 (SEQ ID NO:16); GI PPK2 (SEQ ID NO:17); AA PPK2 (SEQ ID NO:18); TD PPK2 (SEQ ID NO:19); and PF PPK2 (SEQ ID NO:20).

The PNDK has an optimum temperature of 37°C (Motomura et al., Applied and Environmental Microbiology, volume 80, number 8, 2602-2608, 2014). Therefore, use of the PNDK makes it possible to carry out a reaction at relatively low temperature (that is, under moderate conditions). In view of this, the PNDK is therefore preferred as the PPK2 in an embodiment of the present invention.

Furthermore, as described earlier, the PNDK is PPK2 derived from *Pseudomonas aeruginosa.* Therefore, provided that a PPK2 is derived from a microbial species classified in the Pseudomonas genus, this PPK2 can have similar advantages to the foregoing advantages of the PNDK. Thus, a PPK2 derived from a microbial species classified in the Pseudomonas genus is preferred as the PPK2 in an embodiment of the present invention.

Other examples of a microbial species classified in the Pseudomonas genus, other than the foregoing *Pseudomonas aeruginosa* and *Pseudomonas fluorescens,* include the following species: *Pseudomonas oxalaticus, Pseudomonas stuzeri, Pseudomonas chloraphis, Pseudomonas riboflavina, Pseudomonas fragi, Pseudomonas mendocina,* Pseudomonas sp. K-9, *Pseudomonas diminuta, Pseudomonas vesicularis, Pseudomonas caryophylli, Pseudomonas cepacian, Pseudomonas antimicrobica, Pseudomonas plantarii, Pseudomonas marina, Pseudomonas testosterone, Pseudomonas lanceolate, Pseudomonas acidovorans, Pseudomonas rubrisubalbicans, Pseudomonas flava, Pseudomonas palleronii, Pseudomonas pseudoflava, Pseudomonas taeniospiralis, Pseudomonas nautica, Pseudomonas iners, Pseudomonas mesophilica, Pseudomonas radiora, Pseudomonas rhodos, Pseudomonas doudoroffii, Pelomonas saccharophila, Pseudomonas abietaniphila, Pseudomonas alcaligenes, Pseudomonas alcaliphila, Pseudomonas auricularis, Pseudomonas azotoformans, Pseudomonas balearica, Pseudomonas chlororaphis* subsp. *aureofaciens, Pseudomonas chlororaphis* subsp. *chlororaphis, Pseudomonas citronellolis, Pseudomonas cremoricolorata, Pseudomonas flavescens, Pseudomonas fragi, Pseudomonas fulva, Pseudomonas gessardii, Pseudomonas indica, Pseudomonas japonica, Pseudomonas jianii, Pseudomonas jinjuensis, Pseudomonas luteola, Pseudomonas mandelii, Pseudomonas mendocina, Pseudomonas migulae, Pseudomonas monteilii, Pseudomonas mucidolens, Pseudomonas nitroreducens, Pseudomonas nitroreducens* subsp. *thermotolerans, Pseudomonas oleovorans, Pseudomonas oryzihabitans, Pseudomonas parafulva, Pseudomonas pavonaceae, Pseudomonas pertucinogena, Pseudomonas plecoglossicida, Pseudomonas pseudo alcaligenes, Pseudomonas reptilivora, Pseudomonas resinovorans,* Pseudomonas sp., *Pseudomonas straminea, Pseudomonas striafaciens, Pseudomonas syncyanea, Pseudomonas synxantha, Pseudomonas syringae, Pseudomonas taetrolens, Pseudomonas tolaasii, Pseudomonas toyotomiensis, Pseudomonas pickettii, Pseudomonas echinoides, Pseudomonas paucimobilis, Pseudomonas maltophilia,* and *Pseudomonas butanovora.*

In an embodiment of the present invention, the PPK2 may be in the form of (i) a live cell of an organism having the PPK2 activity, (ii) a dead but undamaged cell of an organism having the PPK2 activity, or (iii) a protein that has been isolated from the cell and purified. The degree of purification of the protein that has the PPK2 activity here is not limited to a particular degree, and the purification may be partial purification. The PPK2 may be a freeze-dried or acetone-dried body that has the PPK2 activity, may be the body which has been triturated, or may be a polypeptide itself fixed or a body fixed as-is.
It is preferable not to use live cells having the PPK2 activity. It is more preferable to use neither live cells having the PPK2 activity nor undamaged dead cells.

In an embodiment of the present invention, each of the foregoing ten types of PPK2 may be a protein which (i) has the same amino acid sequence as shown in a corresponding one of SEQ ID NOs: 1 to 10 except that one to several amino acid residues are substituted, deleted inserted and/or added and (ii) has the PPK2 activity (such proteins are hereinafter referred to as proteins of case (a)).

The specific sequence of each protein of case (a) is not limited, provided that the sequence constitutes a protein which (i) is a mutant, a derivative, a variant, an allele, a homologue, an orthologue, a partial peptide, a fusion protein with some other protein/peptide, or the like, each of which is functionally equivalent to a corresponding one of the proteins having the amino acid sequences shown in SEQ ID NOs: 1 to 10 and (ii) has the PPK2 activity. The number of amino acids that may be deleted, substituted or added is not limited, provided that the foregoing function is not impaired, and is intended to mean the number of amino acids that can be deleted, substituted or added by a known insertion method such as site-directed mutagenesis. The number of such amino acids is preferably five or less, more preferably three or less (e.g., three amino acids, two amino acids, or one amino acid). In this specification, the term "mutation" mainly refers to a mutation artificially introduced by, for example, site-directed mutagenesis; however, the term "mutation" may refer to an equivalent naturally-occurring mutation.

In a case where an amino acid residue is substituted, it is preferable that the amino acid residue is substituted with another amino acid whose side chain has the same property. Examples of properties of amino acid side chains include: hydrophobic amino acids (A, I, L, M, F, P, W, Y, V); hydrophilic amino acids (R, D, N, C, E, Q, G, H, K, S, T); amino acids with aliphatic side chain (G, A, V, L, I, P); amino acids with hydroxyl-containing side chain (S, T, Y); amino acids with sulfur-atom-containing side chain (C, M); amino acids with carboxylic-acid-and-amide-containing side chain (D, N, E, Q); amino acids with base-containing side chain (R, K, H); and amino acids with aromatic-containing side chain (H, F, Y, W) (the letters provided in parentheses are each a single letter code of an amino acid). It is known that a polypeptide having a certain amino acid sequence maintains its biological activity even if one to several amino acid residues in the amino acid sequence are deleted, added and/or substituted by some other amino acid and thereby the amino acid sequence is modified. It is preferable that a mutated amino acid residue and the original amino acid residue have as many common properties as possible.

In this specification, the phrase "functionally equivalent" is intended to mean that a certain protein has a biological function and/or a biochemical function equivalent to (identical to and/or similar to) a target protein. Biological properties can include specificity with regard to expression site, expression level, and the like. Whether or not the protein having a mutation(s) introduced therein has a desired function can be determined by (i) obtaining a transformant in which a gene coding for that protein is introduced and expressed and (ii) checking whether this transformant can generate ATP from ADP and PolyP.

In an embodiment of the present invention, each of the foregoing ten types of PPK2 may be a protein which (i) has a sequence homology of not less than 80% with the amino acid sequence shown in a corresponding one of SEQ ID NOs:1 to 10 and (ii) has the PPK2 activity (such proteins are hereinafter referred to as proteins of case (b)).

The specific sequence of each protein of case (b) is not limited, provided that the sequence constitutes a protein which (i) is a mutant, a derivative, a variant, an allele, a homologue, an orthologue, a partial peptide, a fusion protein with some other protein/peptide, or the like, each of which is functionally equivalent to a corresponding one of the proteins having the amino acid sequences shown in SEQ ID NOs: 1 to 10 and (ii) has the PPK2 activity.

The phrase "an amino acid sequence has a homology with another amino acid sequence" means that at least 80%, more preferably not less than 90%, even more preferably not less than 95% (for example, not less than 95%, not less than 96%, not less than 97%, not less than 98%, not less than 99%) of the entire amino acid sequence (or an entire region that is necessary for functional expression) is identical to that of the another amino acid sequence. The homology of an amino acid sequence can be determined with use of a BLASTN program (nucleic acid level) or a BLASTX program (amino acid level) (Altschul et al. J. Mol. Biol., 215: 403-410, 1990). These programs are based on the algorithm BLAST by Karlin and Altschul (Proc. Natl. Acad. Sci. USA, 87:2264-2268, 1990, Proc. Natl. Acad. Sci. USA, 90: 5873-5877, 1993). In a case where a base sequence is analyzed by BLASTN, the parameters employed here are, for example, score = 100 and wordlength = 12. In a case where an amino acid sequence is analyzed by BLASTX, parameters employed here are, for example, score = 50 and wordlength = 3. In a case where an amino acid sequence is analyzed with use of Gapped BLAST program, such an analysis can be carried out as disclosed in Altschul et al. (Nucleic Acids Res. 25: 3389-3402, 1997). In a case of using the BLAST program and the Gapped BLAST program, default parameters of these programs are used. Specific methods of these analyses are known to those skilled in the art. For a comparative base sequence or amino acid sequence to be aligned optimally, addition or deletion (for example, introduction of a gap) may be permitted.

In this specification, the term "homology" is intended to mean the proportion of amino acid residues that have a similar property to those of a comparative sequence (e.g., homology, positive); however, the "homology" is preferably the proportion of amino acid residues that are identical to those of the comparative sequence. That is, the "homology" is preferably "identity". Note that the properties of amino acid sequences have already been discussed earlier.

In an embodiment of the present invention, each of the foregoing ten types of PPK2 may be a protein that is encoded by a gene having a base sequence shown in a corresponding one of SEQ ID NOs:11 to 20 (such proteins are hereinafter referred to proteins of case (c)).

With regard to the proteins of case (c), SEQ ID NOs: 11 to 20 show base sequences (open reading frames: ORFs) of genes coding for proteins having amino acid sequences shown in SEQ ID NOs: 1 to 10, respectively.

In an embodiment of the present invention, in a case where the foregoing ten types of PPK2 are the proteins of case (c), the proteins may be those encoded by genes having modified versions of the respective base sequences of SEQ ID NOs: 11 to 20 which have been modified for, for example, an improvement in expression in a host cell. Each of the proteins of case (c) may have its N-terminus cut off or may have been cleaved at some other position, for the purpose of improvement in expression of the PPK2 in a host cell. Codons may be optimized for the same purpose.

One example of modification of a base sequence is, as shown in Example 3 (described later), to cut off 81 amino acids at the N terminus of wild-type PNDK and substitute alanine at position 82 with methionine which is an initiation codon. Other examples include, as shown in Example 2 (described later): (i) removing amino acids at positions 1 to 85 at the N terminus of an amino acid sequence from native PF PPK2 and introducing a P86M mutation; and (ii) removing amino acids at positions 1 to 86 at the N terminus and introducing a G87M mutation.

The foregoing genes/proteins may be obtained by a usually-used polynucleotide modification method. Specifically, substitution, deletion, insertion and/or addition of a specific base(s) in a polynucleotide that carries genetic information of a protein make it possible to prepare a polynucleotide that carries genetic information of a desired recombinant protein. A specific method of converting a base of a polynucleotide is, for example, use of a commercially-available kit (KOD-Plus Site-Directed Mutagenesis Kit [TOYOBO], Transformer Site-Directed Mutagenesis Kit [Clontech], QuickChange Site Directed Mutagenesis Kit [Stratagene] or the like) or use of a polymerase chain reaction (PCR). These methods are known to those skilled in the art.

Each of the foregoing genes may consist only of a polynucleotide that codes for a corresponding protein, but may have some other base sequence added thereto. The base sequence added is not particularly limited, and examples thereof include: base sequences coding for a label (e.g., histidine tag, Myc tag, FLAG tag, or the like); base sequences coding for a fusion protein (e.g., streptavidin, cytochrome, GST, GFP, MBP or the like): base sequences coding for a promoter sequence; and base sequences coding for a signal sequence (e.g., endoplasmic reticulum translocation signal sequence, secretion sequence, or the like). The site at which any of such base sequences is added is not particularly limited. The base sequence may be added to, for example, a site corresponding to the N terminus or C terminus of a translated protein.

### <3. PPK2 gene>

An embodiment of the present invention provides a PPK2 gene coding for any of the foregoing proteins.

The PPK2 gene may either be a nucleotide composed of a native sequence or a nucleotide composed of an artificially modified sequence. The PPK2 gene is preferably a nucleotide composed of a sequence which has been subjected to codon optimization for expression in a host cell (for example, *E. coli*).

### <4. Vector>

An embodiment of the present invention provides a vector that contains a gene discussed in the <3. PPK2 gene> section. Examples of the vector not only include expression vectors for expressing the gene in a host cell in order to prepare a transformant but also include those which are for use in production of a recombinant protein.

A base vector serving as a base for the above vector can be any of various kinds of commonly-used vectors. Examples include plasmids, phages and cosmids, from which a base vector can be selected appropriately according to a cell to which it is introduced and how it is introduced. That is, the vector is not limited to a specific kind, and any vector that can be expressed in a host cell can be selected as appropriate. An appropriate promoter sequence for unfailingly expressing the gene may be selected according to the type of host cell, and this promoter sequence and the foregoing gene may be incorporated into a plasmid or the like to obtain a vector. Such a vector may be used as the expression vector. Examples of the expression vector that can be employed include: phage vectors, plasmid vectors, viral vectors, retroviral vectors, chromosome vectors, episome vectors, and virus-derived vectors (for example, bacterial plasmids, bacteriophages, yeast episomes, yeast chromosomal elements and viruses [for example, baculovirus, papovavirus, saccinia virus, adenovirus, avipoxvirus, pseudorabies virus, herpesvirus, lentivirus and retrovirus]); and vectors derived from combinations thereof (for example, cosmids and phagemids).

Examples of a vector suitable for use in bacteria include: pQE30, pQE60, pQE70, pQE80 and pQE9 (available from Qiagen); pTipQC1 (available from Qiagen or Hokkaido System Science Co., Ltd.), pTipRT2 (available from Hokkaido System Science Co., Ltd.); pBS vector, Phagescript vector, Bluescript vector, pNH8A, pNH16A, pNH18A and pNH46A (available from Stratagene); ptrc99a, pKK223-3, pKK233-3, pDR540 and pRIT5 (available from Addgene); pRSF (available from MERCK); and pAC (available from NIPPON GENE CO., LTD.). In particular, examples of a vector suitable for use in a case of *E. coli* include pUCN18 (which can be prepared by modifying pUC18 available from Takara Bio Inc.), pSTV28 (available from Takara Bio Inc.), and pUCNT (PCT International Publication No. WO 94/03613).

The insertion of the foregoing gene is preferably such that the gene is operatively linked to an appropriate promoter. The other appropriate promoters can be those known to those skilled in the art, and are not particularly limited. Examples of the promoter include: lacUV5 promoter, trp promoter, trc promoter, tac promoter, lpp promoter, tufB promoter, recA promoter, pL promoter, lacI promoter, lacZ promoter, T3 promoter, T5 promoter, T7 promoter, gap promoter, OmpA promoter, and SV40 early promoter and late promoter; and retrovirus LTR promoter.

The vector preferably further contains sites for transcription initiation and transcription termination and contains, within a transcribed region, a site for ribosome binding for translation. A region coding for a mature transcript expressed by a vector construct contains a transcription initiation AUG at the start of a to-be-translated polypeptide and contains a stop codon positioned appropriately at the end.

A host into which a vector is introduced is not particularly limited. Any of various kinds of cells can be used suitably. Typical examples of an appropriate host include bacteria, yeast, filamentous fungi, plant cells, and animal cells. *E. coli* is particularly preferred. An appropriate culture medium and conditions for the above host cell can be any of those known in this technical field.

A method of introducing the foregoing vector into a host cell, that is, a method of transformation, is not particularly limited. Suitable examples include conventionally known methods such as electroporation, calcium phosphate transfection, liposome transfection, DEAE-dextran transfection, microinjection, cationic lipid-mediated transfection, electroporation, transduction, and infection. Such methods are stated in many standard laboratory manuals such as Basic Methods In Molecular Biology (1986) by Davis et al.

### <5. Transformant>

An embodiment of the present invention provides a transformant that contains a gene discussed in the <3. Gene> section or a recombinant vector discussed in the <4. Vector> section. As used herein, the phrase "contains a gene or a vector" is intended to mean that the gene or vector has been introduced in a target cell (host cell) by a known genetic engineering procedure (gene manipulation technique) such that the gene can be expressed. The meaning of the term "transformant" includes not only cells, tissues, and organs but also living individuals.

The transformant can be prepared (produced) by, for example, transforming an organism with the foregoing vector. The organism to be transformed is not particularly limited, and can be, for example, any of various kinds of organism exemplary listed earlier for the host cell.

A host cell for use in an embodiment of the present invention is not particularly limited, provided that the cell allows the expression of an introduced gene or of a protein encoded by a gene contained in a vector. Examples of a microorganism available for use as a host cell include: bacteria such as those belonging to the genus Escherichia, those belonging to the genus Bacillus, those belonging to the genus Pseudomonas, those belonging to the genus Serratia, those belonging to the genus Brevibacterium, those belonging to the genus Corynebacterium, those belonging to the genus Streptococcus, and those belonging to the genus Lactobacillus; actinomycetes such as those belonging to the genus Rhodococcus and those belonging to the genus Streptomyces; yeast such as those belonging to the genus Saccharomyces, those belonging to the genus Kluyveromyces, those belonging to the genus Schizosaccharomyces, those belonging to the genus Zygosaccharomyces, those belonging to the genus Yarrowia, those belonging to the genus Trichosporon, those belonging to the genus Rhodosporidium, those belonging to the genus Pichia, and those belonging to the genus Candida; and fungi such as those belonging to the genus Neurospora, those belonging to the genus Aspergillus, those belonging to the genus Cephalosporium, and those belonging to the genus Trichoderma. Not only microorganisms but also plant cells, animal cells and the like can be used as a host cell. Among those listed above, a bacterium is preferred in view of introduction and expression efficiency, and *E. coli* is particularly preferred.

### [Production of substance using ATP]

In an embodiment of the present invention, production of a substance using ATP is not particularly limited, provided that the method is to produce a substance at the expense of energy derived from ATP. Examples of production of a substance using ATP include: production of oxidized glutathione, production of reduced glutathione, production of S-adenosylmethionine, production of sugar phosphate, production of acetyl-CoA, production of propanoyl-CoA, production of oxyluciferin, production of guanosine-3'-diphosphate-5'-triphosphate, production of 5-phosphoribosyl-1-pyrophosphate, production of acyl-CoA, production of biotin-CoA, production of aminoacyl-tRNA, production of circular RNA, production of L-asparagine, production of L-asparatic acid, production of sugar nucleotide, and production of 3'-phosphoadenosine-5'-phosphosulfate. It should be easy for those skilled in the art to understand enzymatic reactions that generate a substance using ATP other than the foregoing reactions, by searching, for example, KEGG (http://www.genome.jp/kegg/).

The following description will discuss <1. Method of producing oxidized glutathione> and <2. Method of producing reduced glutathione> which are typical examples of production of a substance using ATP.

### <1. Method of producing oxidized glutathione>

An embodiment of the present invention provides a method of producing a substance, the method including the steps of:
(1) allowing L-glutamic acid and L-cystine to react with each other to produce oxidized γ-glutamylcysteine; and
(2) allowing the oxidized γ-glutamylcysteine obtained from step (1) and glycine to react with each other to produce oxidized glutathione.

In an embodiment of the present invention, a method of producing oxidized glutathione is preferably a method disclosed in PCT International Publication No. WO 2016/002884.

In an embodiment of the present invention, the step (1) is represented by, for example, the following formula.

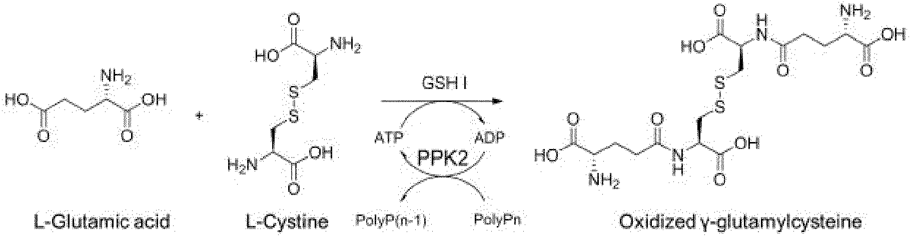

The step (1) includes generating oxidized γ-glutamylcysteine by allowing L-cystine and L-glutamic acid to react with each other in the presence of GSHI and ATP.

The GSHI for use in the step (1) is not particularly limited, provided that the GSHI has the above-described activity. The origin of the GSHI is not particularly limited, and GSHI derived from a microorganism, an animal, a plant, or the like can be used. GSHI derived from a microorganism is preferred. Particularly, for example, those derived from enteric bacteria such as *Escherichia coli,* those derived from bacteria such as coryneform bacteria, thermophilic bacteria/thermotolerant bacteria, psychrophilic bacteria/psychrotolerant bacteria, acidophilic bacteria/aciduric bacteria, basophilic bacteria/base-resistant bacteria, methylotroph, halogen-resistant bacteria, sulfur bacteria, and radiation-resistant bacteria, and those derived from eukaryotic microorganisms such as yeast are preferred.

In an embodiment of the present invention, the step (2) is represented by, for example, the following formula.

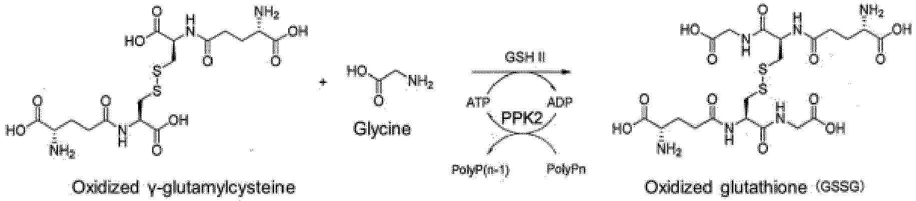

On the contrary, the step (2) includes generating oxidized glutathione by allowing the oxidized γ-glutamylcysteine and glycine to react with each other in the presence of GSHII and ATP.

The GSHII for use in the step (2) is not particularly limited, provided that the GSHII has the foregoing activity. The origin of the GSHII is not particularly limited, and GSHII derived from a microorganism, an animal, a plant, or the like can be used. GSHII derived from a microorganism is preferred. Particularly, for example, those derived from enteric bacteria such as *Escherichia coli,* those derived from bacteria such as coryneform bacteria, thermophilic bacteria/thermotolerant bacteria, psychrophilic bacteria/psychrotolerant bacteria, acidophilic bacteria/aciduric bacteria, basophilic bacteria/base-resistant bacteria, methylotroph, halogen-resistant bacteria, sulfur bacteria, and radiation-resistant bacteria, and those derived from eukaryotic microorganisms such as yeast are preferred.

In an embodiment of the present invention, GSHF may be used instead of one of the GSHI and GSHII or instead of both of the GSHI and GSHII. GSHF is a bifunctional glutathione synthase that has both the functions of the two enzymes GSHI and GSHII, and is not particularly limited, provided that the GSHF can substitute the GSHI and GSHII. The origin of the GSHF is not particularly limited, and GSHF derived from a microorganism, an animal, a plant, or the like can be used. GSHF derived from a microorganism is preferred. Particularly, for example, those derived from enteric bacteria such as *Escherichia coli,* those derived from bacteria such as coryneform bacteria, thermophilic bacteria/thermotolerant bacteria, psychrophilic bacteria/psychrotolerant bacteria, acidophilic bacteria/aciduric bacteria, basophilic bacteria/base-resistant bacteria, methylotroph, halogen-resistant bacteria, sulfur bacteria, radiation-resistant bacteria, and lactic acid bacteria, and those derived from eukaryotic microorganisms such as yeast are preferred. The GSHF is, for example, preferably GSHF derived from at least one selected from the group consisting of: Streptococcus bacteria such as *Streptococcus agalactiae, Streptococcus mutans, Streptococcus suis, Streptococcus thermophilus, Streptococcus sanguinis, Streptococcus gordonii,* and *Streptococcus uberis;* Lactobacillus bacteria such as *Lactobacillus plantarum, Lactobacillus casei, Lactobacillus rhamnosus, Lactobacillus paracasei, Lactobacillus plantarum,* and *Lactobacillus fermentum;* Desulfotalea bacteria such as *Desulfotalea psychrophila;* Clostridium bacteria such as *Clostridium perfringens;* Listeria bacteria such as *Listeria innocua* and *Listeria monocytogenes;* Enterococcus bacteria such as *Enterococcus faecalis, Enterococcus faecium,* and *Enterococcus italicus;* Pasteurella bacteria such as *Pasteurella multocida;* Mannheimia bacteria such as *Mannheimia succiniciprodecens;* Haemophilus bacteria such as *Haemophilus somnus;* Actinobacillus bacteria such as *Actinobacillus succinogenes* and *Actinobacillus pleuropneumoniae;* and Bacillus bacteria such as *Bacillus cereus.*

In an embodiment of the present invention, each of the foregoing ten types of PPK2 functions in a manner coupled with at least one selected from the group consisting of γ-glutamylcysteine synthase (GSHI), glutathione synthase (GSHII), and bifunctional glutathione synthase (GSHF).

A combination of any of the ten types of PPK2 and at least one selected from the group consisting of the GSHI, GSHII, and GSHF is not particularly limited and may be any combination, provided that oxidized glutathione can be produced with a high conversion rate. The GSHI, GSHII, and GSHF may be used in combination with different types of PPK2 or may each be used in combination with the same type of PPK2.

In an embodiment of the present invention, each of the enzymes PPK2, GSHI, GSHII, and GSHF may be (i) in the form of a live cell of an organism having a corresponding enzyme activity, (ii) in the form of a dead but undamaged cell of an organism having a corresopnding enzyme activity, (iii) in the form in which the enzyme is present extracellularly, specifically, in the form of the foregoing cell of an organism which has been triturated, or (iv) in the form of a protein that has been isolated from the cell and purified. It is preferable not to use live cells having the PPK2 activity. It is more preferable to use neither live cells having the PPK2 activity nor undamaged dead cells.

In an embodiment of the present invention, the polyphosphoric acid mixture is preferably used (i.e., added) both in the steps (1) and (2) in view of the rate of conversion to oxidized glutathione; however, the polyphosphoric acid mixture may be used in only one of the steps (1) and (2).

### <2. Method of producing reduced glutathione>

An embodiment of the present invention provides a method of producing a substance, the method including the steps of:
(1) allowing L-glutamic acid and L-cysteine to react with each other to produce γ-glutamylcysteine; and
(2) allowing the γ-glutamylcysteine obtained from step (1) and glycine to react with each other to produce reduced glutathione.

In an embodiment of the present invention, a method of producing reduced glutathione is preferably a method disclosed in PCT International Publication No. WO2016/017631. WO2016/017631 discloses carrying out a reaction in a nitrogen atmosphere in order to prevent the oxidation of reduced glutathione; however, the production of reduced glutathione is not limited to a reaction in a nitrogen atmosphere.

In an embodiment of the present invention, the step (1) is represented by, for example, the following formula.

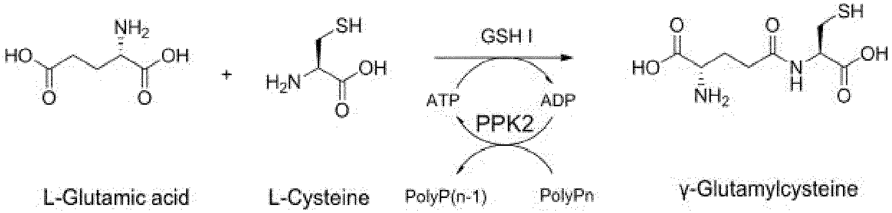

The step (1) includes generating γ-glutamylcysteine by allowing L-cysteine and L-glutamic acid to react with each other in the presence of GSHI and ATP.

The GSHI for use in the step (1) is not particularly limited, provided that the GSHI has the above-described activity. The origin of the GSHI is not particularly limited, and GSHI derived from a microorganism, an animal, a plant, or the like can be used. GSHI derived from a microorganism is preferred. Particularly, for example, those derived from enteric bacteria such as *Escherichia coli,* those derived from bacteria such as coryneform bacteria, and those derived from eukaryotic microorganisms such as yeast are preferred.

In an embodiment of the present invention, the step (2) is represented by, for example, the following formula.

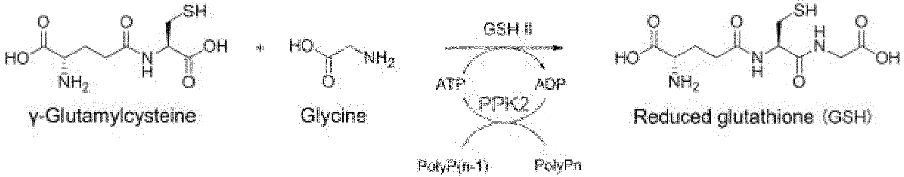

On the contrary, the step (2) includes generating reduced glutathione by allowing the γ-glutamylcysteine and glycine to react with each other in the presence of GSHII and ATP.

The GSHII for use in the step (2) is not particularly limited, provided that the GSHII has the foregoing activity. The origin of the GSHII is not particularly limited, and GSHII derived from a microorganism, an animal, a plant, or the like can be used. GSHII derived from a microorganism is preferred. Particularly, for example, those derived from enteric bacteria such as *Escherichia coli,* those derived from bacteria such as coryneform bacteria, and those derived from eukaryotic microorganisms such as yeast are preferred.

In an embodiment of the present invention, GSHF may be used instead of one of the GSHI and GSHII or instead of both of the GSHI and GSHII. The function, origin, and the like of the GSHF are the same as those described in the <1. Method of producing oxidized glutathione> section.

In an embodiment of the present invention, each of the foregoing ten types of PPK2 functions in a manner coupled with at least one selected from the group consisting of γ-glutamylcysteine synthase (GSHI), glutathione synthase (GSHII), and bifunctional glutathione synthase (GSHF).

A combination of any of the ten types of PPK2 and at least one selected from the group consisting of the GSHI, GSHII, and GSHF is not particularly limited and may be any combination, provided that reduced glutathione can be produced with a high conversion rate. The GSHI, GSHII, and GSHF may be used in combination with different types of PPK2 or may each be used in combination with the same type of PPK2.

In an embodiment of the present invention, each of the enzymes PPK2, GSHI, GSHII, and GSHF may be (i) in the form of a live cell of an organism having a corresponding enzyme activity, (ii) in the form of a dead but undamaged cell of an organism having a corresponding enzyme activity, (iii) in the form in which the enzyme is present extracellularly, specifically, in the form of the foregoing cell of an organism which has been triturated, or (iv) in the form of a protein that has been isolated from the cell and purified. It is preferable not to use live cells having the PPK2 activity. It is more preferable to use neither live cells having the PPK2 activity nor undamaged dead cells.

In an embodiment of the present invention, the polyphosphoric acid mixture is preferably used (i.e., added) both in the steps (1) and (2) in view of the rate of conversion into reduced glutathione; however, the polyphosphoric acid mixture may be used in only one of the steps (1) and (2).

Specifically, aspects of the present invention encompass the following subject matters.
[1] A method of producing a substance using ATP, wherein: ADP is generated from ATP during the method; the method is coupled with an ATP regeneration reaction in which a polyphosphate kinase 2 and polyphosphoric acid are allowed to react with the ADP to regenerate ATP; and the ATP used in the method includes the ATP regenerated by the ATP regeneration reaction, the method including using, as a substrate for the polyphosphate kinase 2, a polyphosphoric acid mixture that contains polyphosphoric acid molecules with a degree of polymerization of not less than 15 in an amount of not less than 48%.
[2] The method as set forth in [1], wherein the amount of the polyphosphoric acid molecules with a degree of polymerization of not less than 15, contained in the polyphosphoric acid mixture, is not less than 50%.
[3] The method as set forth in [1] or [2], wherein the polyphosphate kinase 2 is at least one selected from the group consisting of: polyphosphate kinase 2 derived from *Pseudomonas aeruginosa;* polyphosphate kinase 2 derived from Synechococcus sp. PCC6312; polyphosphate kinase 2 derived from *Corynebacterium efficiens;* polyphosphate kinase 2 derived from *Kineococcus radiotolerans;* polyphosphate kinase 2 derived from *Pannonibacter indicus;* polyphosphate kinase 2 derived from *Deinococcus radiodurans* K1; polyphosphate kinase 2 derived from *Gulbenkiania indica;* polyphosphate kinase 2 derived from *Arthrobactor aurescens* TC1; polyphosphate kinase 2 derived from *Thiobacillus denitrificans* ATCC25259; and polyphosphate kinase 2 derived from *Pseudomonas fluorescens.*
[4] The method as set forth in any of [1] to [3], wherein the polyphosphate kinase 2 functions in a manner coupled with at least one selected from the group consisting of γ-glutamylcysteine synthase, glutathione synthase, and bifunctional glutathione synthase.
[5] The method as set forth in any of [1] to [4], wherein the method is a method of producing oxidized glutathione or a method of producing reduced glutathione.
[6] The method as set forth in [5], wherein: the method is a method of producing oxidized glutathione; and the method includes the steps of: (1) allowing L-glutamic acid and L-cystine to react with each other to produce oxidized γ-glutamylcysteine; and (2) allowing the oxidized γ-glutamylcysteine obtained from step (1) and glycine to react with each other to produce oxidized glutathione.

In addition, it should be noted that configurations described in the above sections can also be applied in other sections as appropriate. The present invention is not limited to the foregoing embodiments, but can be altered by a skilled person in the art within the scope of the claims. The present invention also encompasses, in its technical scope, any embodiment derived by combining technical means disclosed in differing embodiments. The following description will more specifically discuss the present invention with reference to Examples. However, the present invention is not limited to such Examples.

### EXAMPLES

### [Reference Example 1] Construction of expression vector for polyphosphate kinase

In accordance with information disclosed in PCT International Publication No. WO 2006/080313, the following sequence was chemically synthesized at Eurofins Genomics K.K.: a gene sequence which (i) codes for a polypeptide that is the same as polyphosphate kinase (NCBI Reference Sequence: WP_023109529) (amino acid sequence: SEQ ID NO:1, base sequence: SEQ ID NO:11) derived from *Pseudomonas aeruginosa* except that 81 amino acids at the N terminus of the polyphosphate kinase are cut off and alanine at position 82 is substituted with methionine (initiation codon), (ii) which has been subjected to codon optimization so as to fit with an *E. coli* host and (iii) has an Ndel site added at the 5' terminus of the gene sequence and an EcoRI site added at the 3' terminus of the gene sequence. This gene was digested with NdeI and EcoRI, and inserted between NdeI and EcoRI restriction sites downstream of the lac promoter of a plasmid pUCN18 (a plasmid obtained by modifying T at position 185 of pUC18 [produced by Takara Bio Inc.] to A by PCR and thereby destroying the NdeI site and, in addition, modifying GC at positions 471 and 472 to TG and thereby introducing a new NdeI site). In this way, a recombinant vector pPPK was constructed.

### [Reference Example 2] Preparation of recombinant organism that expresses polyphosphate kinase

*E.coli* HB101 competent cells (produced by Takara Bio Inc.) were transformed with the recombinant vector pPPK constructed in Reference Example 1, thereby obtaining a recombinant organism *E.coli* HB101 (pPPK). Furthermore, *E.coli* HB101 competent cells (produced by Takara Bio Inc.) were transformed with pUCN18, thereby obtaining a recombinant organism *E.coli* HB101 (pUCN18).

### [Reference Example 3] Expression of polyphosphate kinase gene in recombinant organism

The two types of recombinant organism (*E.coli* HB101 [pUCN18] and *E.coli* HB101 [pPPK]) obtained in Reference Example 2 were each inoculated into 5 ml of 2×YT medium (1.6% triptone, 1.0% yeast extract, 0.5% sodium chloride, pH7.0) containing 200 µg/ml of ampicillin, and cultured with shaking at 37°C for 24 hours. Each of the culture solutions obtained through the culture was subjected to centrifugation and thereby bacterial bodies were collected, and the bacterial bodies were suspended in 1 ml of 50 mM Tris-HCl buffer (pH8.0). This was homogenized with use of a UH-50 ultrasonic homogenizer (produced by SMT), and then bacterial residues were removed by centrifugation. In this way, cell-free extracts were obtained.

Polyphosphate kinase activity was measured with use of these cell-free extracts. The polyphosphate kinase activity was measured in the following manner. 5 mM sodium metaphosphate (produced by Wako Pure Chemical Corporation), 10 mM ADP disodium salt (produced by Oriental Yeast Co., Ltd.), 70 mM magnesium sulfate (produced by Wako Pure Chemical Corporation), and the cell-free extract were added to 50 mM Tris-HCl buffer (pH8.0), allowed to react at 30°C for 5 minutes, and generated ATP was quantified by HPLC. The enzymatic activity by which 1 µmol of ATP is generated per minute under these reaction conditions was defined as 1 U. The result was that the ATP-generating activity of *E.coli* HB101 (pUCN18) was not more than 5 U/mL.

### [Reference Example 4] Preparation of polyphosphate kinase

The *E.coli* HB101 (pPPK) obtained in Reference Example 2 was inoculated into 5 ml of 2×YT medium (1.6% triptone, 1.0% yeast extract, 0.5% NaCl, pH7.0) containing 200 µg/ml of ampicillin, and cultured with shaking at 37°C for 24 hours. The enzymatic activity was measured by the method discussed in Reference Example 3, and found to be 120 U/mL. Next, bacterial bodies were collected by centrifugation, suspended in 2.5 ml of 50 mM Tris-HCl buffer (pH8.0), and homogenized ultrasonically to obtain an enzyme liquid (polyphosphate kinase liquid).

### [Production Example 1] Production of oxidized glutathione

Oxidized glutathione was produced through the following two steps: step (A) of producing oxidized γ-glutamylcysteine from L-glutamic acid and L-cystine; and step (B) of producing oxidized glutathione from the oxidized γ-glutamylcysteine and glycine (the oxidized γ-glutamylcysteine was produced by a partially modified version of the method disclosed in <Example 1> of PCT International Publication No. WO 2016/002884).

### <Step (A)>

0.3629 g of sodium L-glutamate monohydrate (2.15 mmol), 0.3113 g of L-cystine dihydrochloride (0.99 mmol), 0.7079 g of magnesium sulfate heptahydrate, 0.0583 g of ATP (0.11 mmol), 0.8 g of sodium metaphosphate, and 12 g of distilled water were mixed together, and 0.8 g of 15 wt% aqueous sodium hydroxide solution was used to adjust the pH of the mixture to 7.5. To the resultant solution, 2 g of an *E. coli* K12-derived γ-glutamylcysteine synthase (GSHI) liquid was added, and a polyphosphate kinase liquid was added so that the total PPK2 activity in the reaction liquid would be 20 U/mL, and a reaction was started. The reaction was carried out at a temperature of 30°C for 6 to 8 hours.

Note that the GSHI liquid was prepared in accordance with Test 1 and Test 4 of PCT International Publication No. WO 2016/002884.

The polyphosphate kinase liquid was prepared in the same manner as described in Reference Examples 1 to 4.

### <Step (B)>

Next, 0.19 g of glycine (2.53 mmol), 2 g of glutathione synthase liquid, a predetermined amount of a polyphosphate kinase liquid, 0.21 g of magnesium sulfate heptahydrate, 0.04 g of ATP, and 0.92 g of aqueous sodium metaphosphate solution (36.2 wt%) were added to the above reaction liquid, and a reaction was started. Before the reaction, 1.1 g of 15 wt% aqueous sodium hydroxide solution was used to adjust the pH to 7.5. The reaction was carried out at a temperature of 30°C for 8 hours. Then, the reaction was stopped and the reaction liquid was analyzed.

The GSHII used here is the modified glutathione synthase (V260A) disclosed in Laid-open publication of Japanese Patent Application, *Tokugan,* No. 2016-214073.

The polyphosphate kinase liquid was prepared in the same manner as described in Reference Examples 1 to 4.

### [Example 1] Production of oxidized glutathione using polyphosphoric acid mixture

A plurality of polyphosphoric acid mixtures, each of which would serve as a substrate for a polyphosphate kinase 2 in an ATP-regenerating system, were prepared, and production of oxidized glutathione was carried out. Each polyphosphoric acid mixture was prepared by: synthesizing polyphosphoric acid in accordance with a method usually used in this technical field; and obtaining a mixture containing the polyphosphoric acid. The production of oxidized glutathione was carried out in accordance with the method discussed in Production Example 1.

As a result, it was found that the rate of conversion to oxidized glutathione is high in a case where a specific polyphosphoric acid mixture is used.

In view of this, the polyphosphoric acid mixture, which achieved a high rate of conversion to oxidized glutathione, was analyzed for the degree of polymerization of polyphosphoric acid. The analysis was carried out under the following conditions.

### <Conditions under which analysis was carried out>

- Ion chromatograph
- Model: ICS-2100 produced by Thermo Fisher Scientific
- Columns: IonPac AG11, AS11 (4 mm × 250 mm)
- Eluent: KOH gradient
- Eluent flow rate: 1.0 mL/min.
- Sample injection volume: 25 pL
- Column temperature: 35°C
- Detector: Conductometric detector

The amount for each degree of polymerization was determined by calculating the proportion of the area of a peak relative to the sum (100%) of the areas of all peaks.

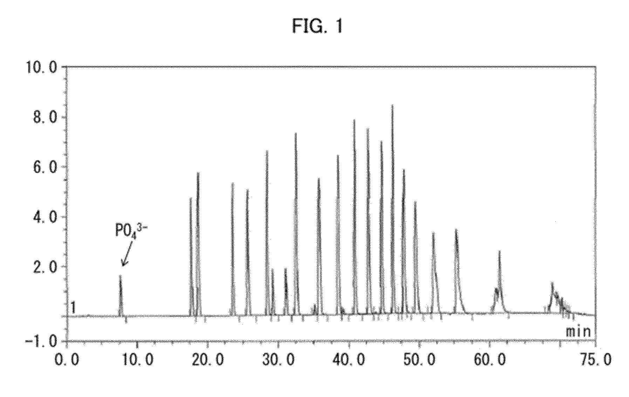

As a result, the distribution of polyphosphoric acid molecules with a high degree of polymerization in the polyphosphoric acid mixture was as follows (see Fig. 1).
- Polyphosphoric acid molecules with a degree of polymerization of not less than 15: not less than 48%
- Polyphosphoric acid molecules with a degree of polymerization of not less than 20: not less than 31%
- Polyphosphoric acid molecules with a degree of polymerization of not less than 36: not less than 4%
- Polyphosphoric acid molecules with a degree of polymerization of not less than 43: not less than 2%
- Polyphosphoric acid molecules with a degree of polymerization of not less than 50: not less than 2%

The results showed that, in a case where such a polyphosphoric acid mixture containing a certain amount or more of polyphosphoric acid molecules with a high degree of polymerization is used as a substrate in a system in which ATP is regenerated by PPK2, it is possible to produce oxidized glutathione with a high conversion rate.

### [Example 2] Enzymatic activity of novel polyphosphate kinases

With regard to the following eight types of polyphosphate kinase (which are inferred from a database search to have polyphosphate kinase activity) and known DR PPK2, polyphosphate kinase liquids were prepared in the same manner as described in Reference Examples 1 to 4, and enzymatic activities were measured in the same manner as described in Reference Example 3.
- PPK2 derived from Synechococcus sp. PCC6312 (Sy PPK2): SEQ ID NO:2
- PPK2 derived from *Corynebacterium efficiens* (CE PPK2): SEQ ID NO:3
- PPK2 derived from *Kineococcus radiotolerans* (KR PPK2): SEQ ID NO:4
- PPK2 derived from *Pannonibacter indicus* (PI PPK2): SEQ ID NO:5
- PPK2 derived from *Deinococcus radiodurans* K1 (DR PPK2): SEQ ID NO:6
- PPK2 derived from *Gulbenkiania indica* (GI PPK2): SEQ ID NO:7
- PPK2 derived from *Arthrobactor aurescens* TC1 (AA PPK2): SEQ ID NO:8
- PPK2 derived from *Thiobacillus denitrificans* ATCC25259 (TD PPK2): SEQ ID NO:9
- PPK2 derived from *Pseudomonas fluorescens* (PF PPK2) (PPK2 obtained by removing amino acids at positions 1 to 85 at the N terminus of native PF PPK2 (SEQ ID NO:10) and introducing a P86M mutation, and PPK2 obtained by removing amino acids at positions 1 to 86 at the N terminus of native PF PPK2 (SEQ ID NO:10) and introducing a G87M mutation)

As a result, all the eight types of novel polyphosphate kinase were found to have enzymatic activity. It was also confirmed that the known DR PPK2 has enzymatic activity.

The enzymatic activity of the PI PPK2 was 138 U/mL. This showed that the enzymatic activity of the PI PPK2 is higher than that of the PNDK (120 U/mL (see Reference Example 4)).

Note that, in a case where the same test as described above was carried out with use of a polyphosphoric acid mixture solution that had been left to stand at room temperature for 13 days from its preparation, the enzymatic activity of the PI PPK2 was 73% of that in a case where a polyphosphoric acid mixture solution immediately after the preparation was used (assuming that the enzymatic activity of this case is 100%).

### [Example 3] Production of oxidized glutathione using various types of polyphosphoric acid mixture

Production of oxidized glutathione was carried out in accordance with the method discussed in Production Example 1.

The following three types of polyphosphate kinase were used (used enzyme was the same between the step (A) and the step (B)).
- PPK2 derived from *Pseudomonas aeruginosa* (PNDK) (a protein obtained by cutting off 81 amino acids at the N terminus of wild-type PNDK (SEQ ID NO:1) and substituting alanine at position 82 with methionine (initiation codon))
- PPK2 derived from *Pannonibacter indicus* (PI PPK2)
- PPK2 derived from Synechococcus sp. PCC6312 (Sy PPK2)

With regard to the PNDK, PI PPK2, and Sy PPK2, tests were carried out under the conditions shown in Table 1 below. The enzymatic activity in a reaction liquid was adjusted by adding a certain amount of culture solution (bacterial bodies) based on the enzymatic activity per culture solution.

**[Table 1]**

| Name of test | Name of enzyme | Duration of storage of metaphosphoric acid |
|---|---|---|
| Test A | PNDK | 0 days (used immediately after preparation) |
| Test B | | 5 days |
| Test C | PI PPK2 | 1 day |
| Test D | | 1 day |
| Test E | | 1 day |
| Test F | | 1 day |
| Test G | | 5 days |
| Test H | | 5 days |
| Test I | Sy PPK2 | 0 days (used immediately after preparation) |
| Test J | | 8 days |

The amount of each polyphosphate kinase liquid added in the step (B) was an amount that achieves a corresponding PPK2 activity in the reaction liquid as shown in Table 2.

**[Table 2]**

| Name of test | Enzymatic activity in reaction liquid (U/mL) |
|---|---|
| Test A | 61 |
| Test B | 86 |
| Test C | 60 |
| Test D | 120 |
| Test E | 47 |
| Test F | 38 |
| Test G | 45 |
| Test H | 22 |
| Test I | 31 |
| Test J | 63 |

On the basis of above, production of oxidized glutathione was carried out. The results are shown in Table 3.

**[Table 3]**

| Name of test | Rate of conversion to oxidized glutathione (%) |
|---|---|
| Test A | 99 |
| Test B | 63 |
| Test C | 99.5 |
| Test D | 99.6 |
| Test E | 99.8 |
| Test F | 99.9 |
| Test G | 57 |
| Test H | 44 |
| Test I | 71 |
| Test J | 22 |

The above results showed that, in cases of all types of polyphosphate kinase, use of an aqueous metaphosphoric acid solution after long-term storage results in a reduction in rate of conversion to oxidized glutathione (this is apparent from a comparison between test A and test B on PNDK, a comparison between tests C-F and tests G-H on PI PPK2, and a comparison between test I and test J on Sy PPK2).

Specifically, the results were as follows: the rate of conversion to oxidized glutathione was lower in cases where an aqueous metaphosphoric acid solution after long-term storage was used than in cases where an aqueous metaphosphoric acid solution after short-term storage was used, although the PPK2 enzymatic activity in the reaction liquid was high in the former case (this was apparent from a comparison between test A and test B on PNDK, a comparison between test F and test G on PI PPK2, and a comparison between test I and test J on Sy PPK2). A reason therefor is inferred to be that, although the PPK2 activity in the reaction system was high enough in tests B, G, and J, the metaphosphoric acid serving as a substrate for polyphosphate kinase was degraded during the storage and became insufficient.

### [Example 4] Changes in composition (degree of polymerization) of polyphosphoric acid mixture: (1)

In view of the results of Example 3, the following test was carried out to confirm that the composition of a polyphosphoric acid mixture changes over time during storage.

Specifically, water was added to sodium metaphosphate to prepare 50 w/v% sodium metaphosphate. This was used as sample 1. After 13 days from the preparation of the sample 1, another 50 w/v% sodium metaphosphate was prepared (sample 2). On the day on which the sample 2 was prepared, the samples 1 and 2 were analyzed for the degree of polymerization of metaphosphoric acid contained therein. The analysis was carried out under the following conditions.

### <Conditions under which analysis was carried out>

- Ion chromatograph
- Model: ICS-2100 produced by Thermo Fisher Scientific
- Columns: IonPac AG11, AS11 (4 mm × 250 mm)
- Eluent: KOH gradient
- Eluent flow rate: 1.0 mL/min.
- Sample injection volume: 25 µL
- Column temperature: 35°C
- Detector: Conductometric detector

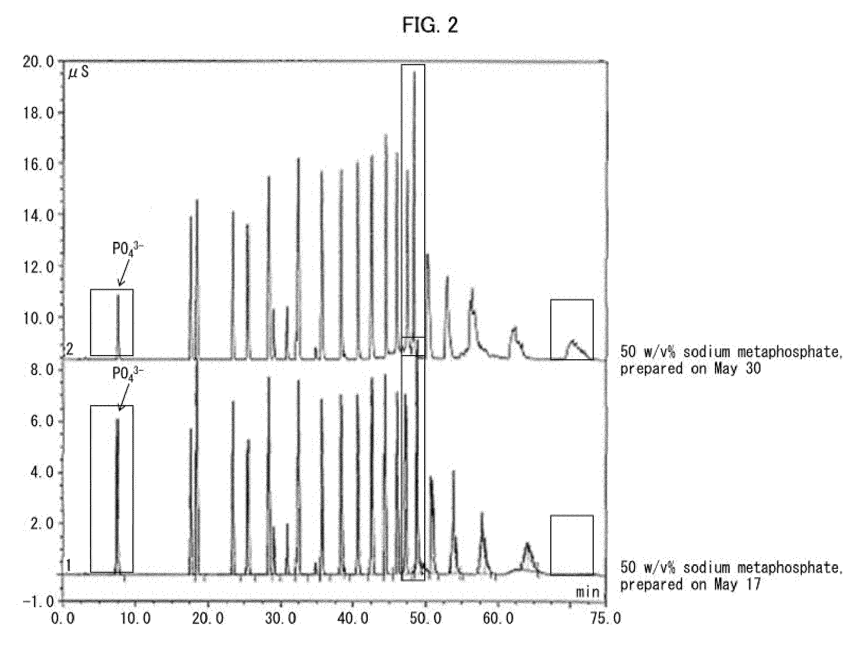

The results are shown in Fig. 2. As is clear from Fig. 2, the sample 1, which had been left to stand for 13 days after the preparation, had a degreased amount of metaphosphoric acid molecules with a high degree of polymerization, as compared to the sample 2 which was analyzed immediately after the preparation. This result shows that, if an aqueous metaphosphoric acid solution is left to stand at room temperature, metaphosphoric acid molecules with a higher degree of polymerization are degraded first.

The combination of the results of this example and the results of Example 3 suggests that, in order to carry out a conversion from ADP to ATP efficiently by polyphosphate kinase, it is not only necessary that the polyphosphate kinase have sufficient level of enzymatic activity but also necessary that metaphosphoric acid serving as a substrate for the polyphosphate kinase be in an appropriate condition (specifically, metaphosphoric acid molecules with a high degree of polymerization be present).

### [Example 5] Changes in composition (degree of polymerization) of polyphosphoric acid mixture: (2)

The following test was carried out to confirm that the composition (degree of polymerization) of polyphosphoric acid mixture changes during production of oxidized glutathione.

Specifically, the same test as test G of Example 3 was carried out, a reaction liquid immediately after the completion of the step (a) and a reaction liquid immediately after the completion of the step (b) were recovered, and the composition (degree of polymerization) of polyphosphoric acid contained in each reaction liquid was checked. The composition (degree of polymerization) was analyzed in accordance with the method discussed in Example 4.

The sample 2 of Example 4 (polyphosphoric acid mixture immediately after preparation) was used as a polyphosphoric acid mixture before consumed (i.e., before used) by PPK2.

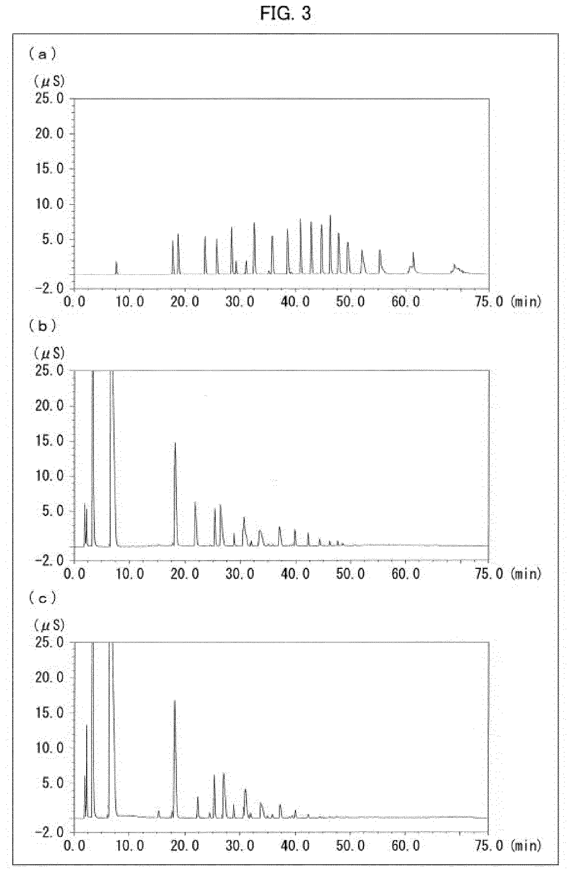

The results are shown in Fig. 3. (a) of Fig. 3 shows the composition (degree of polymerization) of a polyphosphoric acid mixture immediately after preparation, (b) of Fig. 3 shows the composition (degree of polymerization) of a polyphosphoric acid mixture after completion of the step (A), and (c) of Fig. 3 shows the composition (degree of polymerization) of a polyphosphoric acid mixture after completion of the step (B).

The results show that, in the reaction in which ATP is regenerated by PPK2, polyphosphoric acid molecules with a high degree of polymerization are used first.

### Industrial Applicability

The present invention makes it possible to produce a substance using ATP with a high conversion rate at low cost, and is therefore usable in the fields of, for example, production of oxidized glutathione and production of reduced glutathione.

## Claims

1. A method of producing a substance using ATP, wherein:
ADP is generated from ATP during said method;
said method is coupled with an ATP regeneration reaction in which a polyphosphate kinase 2 and polyphosphoric acid are allowed to react with the ADP to regenerate ATP; and
the ATP used in said method includes the ATP regenerated by the ATP regeneration reaction,
said method comprising using, as a substrate for the polyphosphate kinase 2, a polyphosphoric acid mixture that contains polyphosphoric acid molecules with a degree of polymerization of not less than 15 in an amount of not less than 48%.

2. The method as set forth in claim 1, wherein the amount of the polyphosphoric acid molecules with a degree of polymerization of not less than 15, contained in the polyphosphoric acid mixture, is not less than 50%.

3. The method as set forth in claim 1 or 2, wherein the polyphosphate kinase 2 is at least one selected from the group consisting of: polyphosphate kinase 2 derived from *Pseudomonas aeruginosa;* polyphosphate kinase 2 derived from Synechococcus sp. PCC6312; polyphosphate kinase 2 derived from *Corynebacterium efficiens;* polyphosphate kinase 2 derived from *Kineococcus radiotolerans;* polyphosphate kinase 2 derived from *Pannonibacter indicus;* polyphosphate kinase 2 derived from *Deinococcus radiodurans* K1; polyphosphate kinase 2 derived from *Gulbenkiania indica;* polyphosphate kinase 2 derived from *Arthrobactor aurescens* TC1; polyphosphate kinase 2 derived from *Thiobacillus denitrificans* ATCC25259; and polyphosphate kinase 2 derived from *Pseudomonas fluorescens.*

4. The method as set forth in any one of claims 1 to 3, wherein the polyphosphate kinase 2 functions in a manner coupled with at least one selected from the group consisting of γ-glutamylcysteine synthase, glutathione synthase, and bifunctional glutathione synthase.

5. The method as set forth in any one of claims 1 to 4, wherein said method is a method of producing oxidized glutathione or a method of producing reduced glutathione.

6. The method as set forth in claim 5, wherein:
said method is a method of producing oxidized glutathione; and
said method comprises the steps of:
(1) allowing L-glutamic acid and L-cystine to react with each other to produce oxidized γ-glutamylcysteine; and
(2) allowing the oxidized γ-glutamylcysteine obtained from step (1) and glycine to react with each other to produce oxidized glutathione.


SEQUENCE LISTING

<110>  KANEKA CORPORATION

<120>  METHOD OF PRODUCING SUBSTANCE USING ATP

<130>  B170312

<150>  JP 2017-091451
<151>  2017-05-01

<160>  20

<170>  PatentIn version 3.5

<210>  1
<211>  357
<212>  PRT
<213>  Pseudomonas aeruginosa

<400>  1

Met Ser Glu Glu Pro Thr Val Ser Pro Pro Ser Pro Glu Gln Pro Ala
1               5                   10                  15

Ala Gln Pro Ala Lys Pro Ala Arg Pro Ala Ala Arg Arg Ala Pro Arg
            20                  25                  30

Lys Pro Ala Thr Arg Arg Pro Arg Val Ala Ser Pro Ala Gln Lys Ala
        35                  40                  45

Arg Glu Glu Ile Gln Ala Ile Ser Gln Lys Pro Val Ala Leu Gln Val
    50                  55                  60

Ala Ser Ala Pro His Gly Ser Ser Glu Asp Ser Thr Ser Ala Ser Leu
65                  70                  75                  80

Pro Ala Asn Tyr Pro Tyr His Thr Arg Met Arg Arg Asn Glu Tyr Glu
                85                  90                  95

Lys Ala Lys His Asp Leu Gln Ile Glu Leu Leu Lys Val Gln Ser Trp
            100                 105                 110

Val Lys Glu Thr Gly Gln Arg Val Val Val Leu Phe Glu Gly Arg Asp
        115                 120                 125

Ala Ala Gly Lys Gly Gly Thr Ile Lys Arg Phe Met Glu His Leu Asn
    130                 135                 140

Pro Arg Gly Ala Arg Ile Val Ala Leu Glu Lys Pro Ser Ser Gln Glu
145                 150                 155                 160

Gln Gly Gln Trp Tyr Phe Gln Arg Tyr Ile Gln His Leu Pro Thr Ala

165                 170                 175

Gly Glu Met Val Phe Phe Asp Arg Ser Trp Tyr Asn Arg Ala Gly Val
            180                 185                 190

Glu Arg Val Met Gly Phe Cys Ser Pro Leu Gln Tyr Leu Glu Phe Met
        195                 200                 205

Arg Gln Ala Pro Glu Leu Glu Arg Met Leu Thr Asn Ser Gly Ile Leu
    210                 215                 220

Leu Phe Lys Tyr Trp Phe Ser Val Ser Arg Glu Glu Gln Leu Arg Arg
225                 230                 235                 240

Phe Ile Ser Arg Arg Asp Asp Pro Leu Lys His Trp Lys Leu Ser Pro
                245                 250                 255

Ile Asp Ile Lys Ser Leu Asp Lys Trp Asp Asp Tyr Thr Ala Ala Lys
            260                 265                 270

Gln Ala Met Phe Phe His Thr Asp Thr Ala Asp Ala Pro Trp Thr Val
        275                 280                 285

Ile Lys Ser Asp Asp Lys Lys Arg Ala Arg Leu Asn Cys Ile Arg His
    290                 295                 300

Phe Leu His Ser Leu Asp Tyr Pro Asp Lys Asp Arg Arg Ile Ala His
305                 310                 315                 320

Glu Pro Asp Pro Leu Leu Val Gly Pro Ala Ser Arg Val Ile Glu Glu
                325                 330                 335

Asp Glu Lys Val Tyr Ala Glu Ala Ala Ala Ala Pro Gly His Ala Asn
            340                 345                 350

Leu Asp Ile Pro Ala
        355

<210>  2
<211>  296
<212>  PRT
<213>  Synechococcus sp.

<400>  2

Met Ala Glu Leu Asp Ile Thr Ala Ala Pro Leu Glu Ala Gln Thr Glu
1               5                   10                  15

Gly Pro Gly Lys Lys Lys Lys Ala Lys Asp Lys Lys Lys Ala Leu Pro

20                  25                  30

Glu Thr Pro Lys Pro Ser Lys Leu Asp Arg Asp Phe Tyr Asp Lys Glu
        35                  40                  45

Leu Ala Arg Leu Gln Val Glu Leu Val Lys Met Gln Tyr Trp Val Lys
    50                  55                  60

His Ala Gly Leu Lys Ile Val Ile Ile Phe Glu Gly Arg Asp Ala Ala
65                  70                  75                  80

Gly Lys Gly Gly Met Ile Lys Arg Ile Ser Ala Pro Leu Asn Pro Arg
                85                  90                  95

Gly Cys Arg Ile Val Ala Leu Gly Thr Pro Ser Asp Arg Glu Lys Thr
            100                 105                 110

Gln Trp Tyr Phe Gln Arg Tyr Val Glu His Leu Pro Gly Ala Gly Glu
        115                 120                 125

Ile Val Met Phe Asp Arg Ser Trp Tyr Asn Arg Ala Gly Val Glu Trp
    130                 135                 140

Val Met Gly Phe Cys Thr Glu Ala Gln Tyr Asn Glu Phe Met Asp Ser
145                 150                 155                 160

Cys Pro Gln Phe Glu Arg Met Leu Val Lys Ser Gly Ile Ile Leu Ile
                165                 170                 175

Lys Tyr Trp Phe Ser Val Ser Asp Asp Glu Gln Glu Arg Arg Phe Gln
            180                 185                 190

Ala Arg Ile Leu Glu Pro Ala Lys Arg Trp Lys Ile Ser Pro Met Asp
        195                 200                 205

Ile Glu Ser Arg Asp Arg Trp Val Asp Tyr Ser Lys Ala Lys Asp Ala
    210                 215                 220

Met Leu Ala His Thr Asn Ile Pro Glu Ala Pro Trp Phe Thr Val Glu
225                 230                 235                 240

Ala Asp Asp Lys Arg Arg Ala His Leu Asn Cys Ile Ser His Leu Leu
                245                 250                 255

Ser Lys Ile Pro Tyr Glu Asp Ile Thr Pro Pro Ala Ile Asp Leu Pro
            260                 265                 270

Pro Arg Arg Pro Ala Pro Glu Asp Tyr Val Arg Pro Pro Ile Asn Glu
        275                 280                 285

Gln Phe Phe Val Pro Ser Ile Tyr
    290                 295

<210>  3
<211>  351
<212>  PRT
<213>  Corynebacterium efficiens

<400>  3

Met Asn Lys Met Glu Asn Ala Pro Met Pro Thr Phe Gly Lys Glu Leu
1               5                   10                  15

Pro Lys Leu Asp Asn Lys Ala Tyr Lys Lys Glu Leu Lys Arg Leu Gln
            20                  25                  30

Ala Glu Leu Val Glu Met Gln Gln Trp Val Val Glu Thr Gly Thr Arg
        35                  40                  45

Val Val Ile Val Met Glu Gly Arg Asp Ala Ala Gly Lys Gly Ser Ala
    50                  55                  60

Ile Lys Arg Ile Thr Gln Tyr Leu Asn Pro Arg Thr Ala Arg Ile Glu
65                  70                  75                  80

Ala Leu Pro Thr Pro Thr Ser Arg Glu Lys Gly Gln Trp Tyr Phe Gln
                85                  90                  95

Arg Tyr Val Glu Lys Leu Pro Ala Ala Gly Glu Ile Val Ile Phe Asp
            100                 105                 110

Arg Ser Trp Tyr Asn Arg Ala Gly Val Glu Arg Val Met Gly Phe Cys
        115                 120                 125

Thr Ser Gln Glu Tyr Arg Arg Phe Leu His Gln Ala Pro Ile Phe Glu
    130                 135                 140

Arg Leu Leu Val Glu Asp Gly Ile His Leu Arg Lys Tyr Trp Phe Ser
145                 150                 155                 160

Val Ser Asp Glu Glu Gln Leu Ala Arg Phe His Ser Arg Leu Ser Asp
                165                 170                 175

Pro Leu Arg Arg Trp Lys Leu Ser Thr Ile Asp Leu His Ser Ile Thr
            180                 185                 190

Arg Trp Glu Asp Tyr Ser Arg Ala Lys Asp Glu Met Phe Ile His Thr
        195                 200                 205

Asp Ile Pro Ser Ala Pro Trp Tyr Thr Val Glu Ser Glu Glu Lys Lys
    210                 215                 220

Arg Ser Arg Ile Asn Val Ile Ser His Ile Leu Ser Thr Ile Pro Tyr
225                 230                 235                 240

Glu Lys Ile Asp Arg Pro Leu Pro Glu Ile Pro Glu Arg Pro Val Arg
                245                 250                 255

Glu Gly Glu Tyr Ile Arg Pro Pro Arg Asn Glu Phe Arg Tyr Val Pro
            260                 265                 270

Asp Val Ala Ala Cys Leu Glu Glu His Arg Val Ala Ala Ala Arg Glu
        275                 280                 285

Lys Ala Lys Ala Glu Ala Lys Ala Arg Glu Glu Ala Glu Arg Ala Leu
    290                 295                 300

Ala Ala Glu Lys Val Lys Ala Ala Lys Lys Ala Lys Lys Ile Arg Lys
305                 310                 315                 320

Ala Gln Lys Ala Lys Ala Ala Lys Lys Ala Lys Lys Ala Ala Gly Lys
                325                 330                 335

Ala Lys Ala Val Lys Lys Thr Gly Lys Ser Gly Lys Gly Gly Lys
            340                 345                 350

<210>  4
<211>  296
<212>  PRT
<213>  Kineococcus radiotolerans

<400>  4

Met Pro His Val Gln Leu Thr Pro Asp Leu Gly Met Thr Val Arg Asp
1               5                   10                  15

Asp Glu Asp Glu Pro Glu Leu Leu Thr Pro Asp Gly Asn Val Val Asp
            20                  25                  30

Thr Trp Arg Glu Asp Tyr Pro Tyr Asp Glu Arg Leu Asp Arg Lys Glu
        35                  40                  45

Tyr Asp Ala Glu Lys Arg Leu Leu Gln Ile Glu Leu Leu Lys Leu Gln
    50                  55                  60

Arg Trp Leu Lys Ala Ser Gly Glu Arg Ile Val Val Leu Cys Glu Gly
65                  70                  75                  80

Arg Asp Ala Ala Gly Lys Gly Gly Thr Ile Lys Arg Phe Met Glu His
                85                  90                  95

Leu Asn Pro Arg Gly Ala Arg Val Val Ala Leu Glu Lys Pro Ser Glu
            100                 105                 110

Arg Glu Ser Thr Gln Trp Tyr Phe Gln Arg Tyr Val Gln His Leu Pro
        115                 120                 125

Ala Ala Gly Glu Phe Val Leu Phe Asp Arg Ser Trp Tyr Asn Arg Ala
    130                 135                 140

Gly Val Glu Arg Val Met Gly Phe Ala Ser Pro Ala Glu Tyr Asp Arg
145                 150                 155                 160

Phe Val Ala Gln Ala Pro Leu Phe Glu Lys Met Leu Val Asp Asp Gly
                165                 170                 175

Ile His Leu Val Lys Phe Trp Phe Ser Val Thr Arg Ala Glu Gln Arg
            180                 185                 190

Thr Arg Phe Leu Ile Arg Gln Ile Asp Pro Val Arg Gln Trp Lys Leu
        195                 200                 205

Ser Pro Met Asp Leu Glu Ser Leu Asp Arg Trp Asp Glu Tyr Thr Ala
    210                 215                 220

Ala Lys Glu Ala Met Phe Ala Thr Thr Asp Thr Asp Val Ala Pro Trp
225                 230                 235                 240

Thr Val Val Lys Thr Asn Asp Lys Lys Arg Ala Arg Leu Ala Ala Met
                245                 250                 255

Arg His Val Leu Ala Arg Phe Asp Tyr Asp Gly Lys Asp Pro Glu Val
            260                 265                 270

Val Gly Val Pro Asp Pro Leu Leu Val Val His Ala Arg Thr Ile Leu
        275                 280                 285

Glu Ala Asp Arg Arg Pro Ala Ser
    290                 295

<210>  5
<211>  367
<212>  PRT

<213>  Pannonibacter indicus

<400>  5

Met Asp Asp Arg Thr Val Thr Arg Lys Pro Arg Gly Thr Arg Pro Ala
1               5                   10                  15

Pro Ala Ala Asp Ala Val Leu Pro Val Thr Ala Asp Ser Ala Glu Ala
            20                  25                  30

Glu Ala Thr Pro Ser Gly Val Ala Glu Thr Pro Ala Glu Ala Val Thr
        35                  40                  45

Ala Pro Gln Pro Gly Thr Glu Pro Val Ala Ala Pro Glu Ala Ala Leu
    50                  55                  60

Glu Pro Ala Ile Ala Pro Val Ala Ala Ala Pro Arg Lys Thr Leu Ala
65                  70                  75                  80

Glu Met Arg His Asp Pro Ala Ala Ile His Glu Leu Phe Glu Ser Gly
                85                  90                  95

Lys Tyr Pro Tyr Ala Thr Pro Met Arg Arg Ala Pro Tyr Glu Gln Arg
            100                 105                 110

Lys Ala Lys Leu Gln Ala Glu Leu Leu Lys Ala Gln Arg Trp Ile Lys
        115                 120                 125

Glu Thr Gly Gln Arg Val Val Ile Leu Phe Glu Gly Arg Asp Ala Ala
    130                 135                 140

Gly Lys Gly Gly Thr Ile Lys Arg Phe Met Glu His Leu Asn Pro Arg
145                 150                 155                 160

Gly Ala Thr Val Val Ala Leu Gln Lys Pro Thr Glu Gln Glu Ala Ser
                165                 170                 175

Gln Trp Tyr Phe Gln Arg Tyr Ile Asn His Leu Pro Ser Gly Gly Glu
            180                 185                 190

Met Val Leu Phe Asp Arg Ser Trp Tyr Asn Arg Ala Gly Val Glu Arg
        195                 200                 205

Val Met Gly Phe Cys Ser Ala Ser Gln Tyr Leu Glu Phe Met Arg Gln
    210                 215                 220

Cys Pro Glu Ile Glu Arg Met Met Val Arg Asp Gly Ile Arg Leu Phe
225                 230                 235                 240

Lys Tyr Trp Phe Ser Val Ser Arg Glu Glu Gln Arg Arg Arg Phe Met
                245                 250                 255

Glu Arg Gln Thr Asp Pro Leu Lys Gln Trp Lys Leu Ser Pro Ile Asp
            260                 265                 270

Ile Ala Ser Leu Asp Lys Trp Asp Asp Tyr Thr Glu Ala Lys Glu Ala
        275                 280                 285

Met Phe Phe Tyr Thr Asp Thr Ala Asp Ala Pro Trp Thr Ile Val Lys
    290                 295                 300

Ser Asp Asp Lys Lys Arg Ala Arg Leu Asn Cys Met Glu His Phe Leu
305                 310                 315                 320

His Ser Leu Pro Tyr Pro Asp Lys Asp Ile His Leu Val Gly Val Pro
                325                 330                 335

Asp Pro Leu Ile Val Gly Ala Ala His His Val Ile Ser His Asp Thr
            340                 345                 350

His Ile Leu Gly Lys Ala Leu His Pro Glu Met Lys Pro Ala Gly
        355                 360                 365

<210>  6
<211>  266
<212>  PRT
<213>  Deinococcus radiodurans

<400>  6

Met Asp Ile Asp Asn Tyr Arg Val Lys Pro Gly Lys Arg Val Lys Leu
1               5                   10                  15

Ser Asp Trp Ala Thr Asn Asp Asp Ala Gly Leu Ser Lys Glu Glu Gly
            20                  25                  30

Gln Ala Gln Thr Ala Lys Leu Ala Gly Glu Leu Ala Glu Trp Gln Glu
        35                  40                  45

Arg Leu Tyr Ala Glu Gly Lys Gln Ser Leu Leu Leu Ile Leu Gln Ala
    50                  55                  60

Arg Asp Ala Ala Gly Lys Asp Gly Ala Val Lys Lys Val Ile Gly Ala
65                  70                  75                  80

Phe Asn Pro Ala Gly Val Gln Ile Thr Ser Phe Lys Gln Pro Ser Ala
                85                  90                  95

Glu Glu Leu Ser His Asp Phe Leu Trp Arg Ile His Gln Lys Ala Pro
            100                 105                 110

Ala Lys Gly Tyr Val Gly Val Phe Asn Arg Ser Gln Tyr Glu Asp Val
        115                 120                 125

Leu Val Thr Arg Val Tyr Asp Met Ile Asp Asp Lys Thr Ala Lys Arg
    130                 135                 140

Arg Leu Glu His Ile Arg His Phe Glu Glu Leu Leu Thr Asp Asn Ala
145                 150                 155                 160

Thr Arg Ile Val Lys Val Tyr Leu His Ile Ser Pro Glu Glu Gln Lys
                165                 170                 175

Glu Arg Leu Gln Ala Arg Leu Asp Asn Pro Gly Lys His Trp Lys Phe
            180                 185                 190

Asn Pro Gly Asp Leu Lys Asp Arg Ser Asn Trp Asp Lys Phe Asn Asp
        195                 200                 205

Val Tyr Glu Asp Ala Leu Thr Thr Ser Thr Asp Asp Ala Pro Trp Tyr
    210                 215                 220

Val Val Pro Ala Asp Arg Lys Trp Tyr Arg Asp Leu Val Leu Ser His
225                 230                 235                 240

Ile Leu Leu Gly Ala Leu Lys Asp Met Asn Pro Gln Phe Pro Ala Ile
                245                 250                 255

Asp Tyr Asp Pro Ser Lys Val Val Ile His
            260                 265

<210>  7
<211>  350
<212>  PRT
<213>  Gulbenkiania indica

<400>  7

Met Asn Glu Lys Pro Leu Ile Pro Ala Pro Thr Asp Glu Thr Ala Ala
1               5                   10                  15

Ser Ser Glu Gln Ala Ala Pro Asp Thr Pro Ala Ala Ala Ala Ala Ser
            20                  25                  30

Ala Arg Ser Ala Arg Ser Arg Arg Arg Pro Pro Thr Glu Thr Ser
        35                  40                  45

Lys Ala His Asp Glu Ser Val Gln Ala Ile Glu Ala Ala Gln Pro Gly
    50                  55                  60

Pro Val Ala Leu Glu Val Ala Leu Ala Pro Gly Gly Ser Thr Glu Asp
65                  70                  75                  80

Ser Thr Thr Ala Pro Leu Pro Ala Cys Tyr Pro Tyr Arg Thr Arg Met
                85                  90                  95

Arg Arg Pro Glu Tyr Glu Arg Leu Lys Ala Glu Leu Gln Ile Glu Leu
            100                 105                 110

Leu Lys Val Gln Asn Trp Ile Lys Glu Thr Gly Gln Arg Val Ile Val
        115                 120                 125

Leu Phe Glu Gly Arg Asp Ala Ala Gly Lys Gly Gly Thr Ile Lys Arg
    130                 135                 140

Phe Met Glu His Leu Asn Pro Arg Gly Ala Arg Val Val Ala Leu Glu
145                 150                 155                 160

Lys Pro Thr Glu Val Glu Arg Gly Gln Trp Tyr Phe Gln Arg Tyr Ile
                165                 170                 175

Gln His Phe Pro Thr Ala Gly Glu Ile Val Phe Phe Asp Arg Ser Trp
            180                 185                 190

Tyr Asn Arg Ala Gly Val Glu Arg Val Met Gly Phe Cys Thr Pro Asn
        195                 200                 205

Glu Tyr Leu Glu Phe Met Arg Gln Ala Pro Glu Leu Glu Arg Met Leu
    210                 215                 220

Val Asn Ser Gly Ile Arg Leu Phe Lys Phe Trp Phe Ser Val Ser Arg
225                 230                 235                 240

Glu Glu Gln Leu Arg Arg Phe Ile Ala Arg Arg Asp Asp Pro Leu Lys
                245                 250                 255

His Trp Lys Leu Ser Pro Ile Asp Ile Gln Ser Leu Asp Lys Trp Asp
            260                 265                 270

Glu Tyr Thr Ala Ala Lys Gln Ser Met Phe Phe His Thr Asp Thr Ala
        275                 280                 285

Asp Ala Pro Trp Thr Val Ile Lys Ser Asp Asp Lys Lys Arg Ala Arg
    290                 295                 300

Ile Asn Cys Ile Arg His Phe Leu His Gln Leu Pro Tyr Pro Asp Lys
305                 310                 315                 320

Asn Pro Arg Val Ala Cys Gln Pro Asp Pro Leu Leu Val Gly Asn Ala
                325                 330                 335

Ser Lys Val Leu Glu Pro His Glu Thr Gln Val Leu Thr Phe
            340                 345                 350

<210>  8
<211>  314
<212>  PRT
<213>  Arthrobacter aurescens

<400>  8

Met Thr Glu Ala Ser Pro Ser Ser Pro Thr Gly Thr Ser Leu Asp Asp
1               5                   10                  15

Trp Trp Val Arg Asp Asn Leu Arg Glu Thr Ile Asp His Leu Val Glu
            20                  25                  30

Leu Gly Tyr Thr Ile Ser Gly Gly Gln Gly Glu Asp Pro Asp Leu Ile
        35                  40                  45

Asp Pro Gly Gly Ser Ala Val Glu Thr Trp Asn Glu Asp Tyr Pro Tyr
    50                  55                  60

Gln Gln Arg Met Thr Arg Asp Glu Tyr Glu Ile Glu Lys Tyr Arg Leu
65                  70                  75                  80

Gln Ile Glu Leu Leu Lys Phe Gln Tyr Trp Gly Gln Asp Leu Gly Leu
                85                  90                  95

Lys His Val Ile Val Phe Glu Gly Arg Asp Ala Ala Gly Lys Gly Gly
            100                 105                 110

Thr Ile Lys Arg Phe Thr Glu His Leu Asp Pro Arg Ser Ala Arg Thr
        115                 120                 125

Val Ala Leu Ala Lys Pro Ser Asp Arg Glu Gln Gly Gln Trp Tyr Phe
    130                 135                 140

Gln Arg Tyr Ile Gln Gln Phe Pro Thr Ala Gly Glu Ile Val Met Phe
145                 150                 155                 160

Asp Arg Ser Trp Tyr Asn Arg Ala Asn Val Glu Arg Val Met Gly Phe
                165                 170                 175

Cys Thr Asp Asp Glu Tyr Asp Thr Phe Met Gly Gln Ala Pro Val Phe
            180                 185                 190

Glu Lys Met Leu Val Asp Ala Gly Ile His Val Thr Lys Phe Trp Phe
        195                 200                 205

Ser Val Thr Arg Gln Glu Gln Arg Thr Arg Phe Ala Ile Arg Gln Ile
    210                 215                 220

Asp Pro Val Arg Arg Trp Lys Leu Ser Pro Met Asp Leu Ala Ser Leu
225                 230                 235                 240

Asp Arg Trp Asp Glu Tyr Thr Asp Ala Lys Glu Arg Thr Phe Leu His
                245                 250                 255

Thr Asp Ser Asp His Ala Pro Trp Ile Thr Ile Lys Ser Asn Asp Lys
            260                 265                 270

Lys Arg Ala Arg Ile Asn Ala Met Arg Tyr Phe Leu Asn Gln Phe Asp
        275                 280                 285

Tyr Glu Asp Lys Asp Thr Ser Val Val Tyr Asp Ala Asp Pro Leu Ile
    290                 295                 300

Leu Arg Arg Gly Arg Asp Ala Val Gly Asp
305                 310

<210>  9
<211>  269
<212>  PRT
<213>  Thiobacillus denitrificans

<400>  9

Met Lys Pro Arg Asp Thr Arg Val Lys Pro Gly Ser Arg Val Ser Leu
1               5                   10                  15

Ala Asp Trp His Thr Asp Gly Asp Ala Phe Val Gly Lys Asp Lys Ala
            20                  25                  30

Ala Ser Met Ala Arg Leu Asp Asp Asp Arg Val Arg Leu Glu Glu Leu
        35                  40                  45

Gln Glu Leu Leu Tyr Ala Glu Gly Lys His Arg Leu Leu Val Val Leu
    50                  55                  60

Gln Ala Met Asp Thr Ala Gly Lys Asp Ser Thr Ile Arg His Val Phe
65                  70                  75                  80

Arg Gly Val Asp Pro Leu Gly Val Arg Val Ala Asn Phe Gly Val Pro
                85                  90                  95

Ser Thr His Glu Leu Arg His Asp Tyr Leu Trp Arg Val His Pro His
            100                 105                 110

Val Pro Ala Ser Gly Glu Ile Ala Ile Phe Asn Arg Ser His Tyr Glu
        115                 120                 125

Asp Val Leu Val Pro Arg Val Asn Gly Ala Ile Gly His Ala Glu Cys
    130                 135                 140

Glu Arg Arg Tyr Arg Gln Ile Asn Asp Phe Glu Arg Met Leu Ser Glu
145                 150                 155                 160

Thr Gly Thr Thr Ile Arg Lys Phe Tyr Leu His Ile Ser Lys Asp Glu
                165                 170                 175

Gln Lys Lys Arg Leu Glu Ala Arg Arg Asp Thr Pro Lys Lys Arg Trp
            180                 185                 190

Lys Phe Gln Pro Gly Asp Leu Ala Val Arg Ala Gln Trp Asp Asp Tyr
        195                 200                 205

Arg Ala Ala Tyr Asp Ala Leu Leu Ser Ala Thr Ser Thr Arg His Ala
    210                 215                 220

Pro Trp His Val Val Pro Ala Asp Asp Lys Leu Ala Arg Asn Leu Ile
225                 230                 235                 240

Val Ser Ala Leu Leu Ile Glu Ala Leu Glu Gly Leu Asp Met Arg Tyr
                245                 250                 255

Pro Glu Pro Val Ala Gly Val Ala Gly Thr Pro Ile Ile
            260                 265

<210>  10
<211>  362
<212>  PRT
<213>  Pseudomonas fluorescens

<400>  10

Met Ser Glu Glu Ser Thr Ala Leu Pro Leu Pro Pro Ala Pro Val Gln
1               5                   10                  15

Lys Ala Pro Ala Ser Thr Ser Pro Ala Ser Thr Lys Ala Ala Pro Arg
            20                  25                  30

Lys Ala Ala Thr Pro Arg Pro Arg Arg Pro Arg Thr Thr Lys Ala Ala
        35                  40                  45

Pro Val Lys Ala Ala Glu Ala Glu Ile Ser Ala Ile Ser Gln Lys Pro
    50                  55                  60

Met Ala Leu Gln Val Ala Asn Ala Pro Arg Gly Ser Asn Glu Asp Ser
65                  70                  75                  80

Val Ser Ala Ala Leu Pro Gly Asn Tyr Pro Tyr Arg Asn Arg Met Arg
                85                  90                  95

Arg Ala Glu Tyr Glu Lys Ala Lys Asn Glu Leu Gln Ile Glu Leu Leu
            100                 105                 110

Lys Val Gln Ser Trp Val Lys Glu Thr Gly Gln Arg Ile Val Val Leu
        115                 120                 125

Phe Glu Gly Arg Asp Ala Ala Gly Lys Gly Gly Thr Ile Lys Arg Phe
    130                 135                 140

Met Glu His Leu Asn Pro Arg Gly Ala Arg Ile Val Ala Leu Glu Lys
145                 150                 155                 160

Pro Ser Glu Gln Glu Lys Gly Gln Trp Tyr Phe Gln Arg Tyr Ile Gln
                165                 170                 175

His Leu Pro Thr Ala Gly Glu Met Val Phe Phe Asp Arg Ser Trp Tyr
            180                 185                 190

Asn Arg Ala Gly Val Glu Arg Val Met Glu Phe Cys Ser Pro Leu Gln
        195                 200                 205

Tyr Leu Glu Phe Met Arg Gln Thr Pro Glu Leu Glu Arg Met Leu Cys
    210                 215                 220

Asn Ser Gly Ile Leu Met Phe Lys Phe Trp Phe Ser Val Asn Arg Glu
225                 230                 235                 240

Glu Gln Leu Arg Arg Phe Ile Ser Arg Arg Asp Asp Pro Leu Lys His
                245                 250                 255

Trp Lys Leu Ser Pro Ile Asp Ile Lys Ser Leu Asp Lys Trp Asp Glu
            260                 265                 270

Tyr Thr Ala Ala Lys Gln Ala Met Phe Phe His Thr Asp Thr Ala Asp

275                 280                 285

Ala Pro Trp Thr Val Ile Lys Ser Asp Asp Lys Lys Arg Ala Arg Ile
    290                 295                 300

Asn Cys Ile Arg His Phe Leu His Glu Leu Asp Tyr Pro Gly Lys Asp
305                 310                 315                 320

Leu Lys Val Ala His Ala Pro Asp Pro Leu Leu Val Gly Arg Ala Ser
                325                 330                 335

Arg Gly Leu Glu Glu Asp Glu Arg Thr Gln Ala Gln Ala Ala Thr Asp
            340                 345                 350

Ala Gly Ala Thr Lys Leu Ala Leu Ser Ala
        355                 360

<210>  11
<211>  1074
<212>  DNA
<213>  Pseudomonas aeruginosa

<400>  11
atgagcgaag aacccactgt cagtcccccc tcccccgagc aacccgccgc gcagccggcc      60

aagccggccc ggccagccgc ccgccgcgcc ccgcgcaagc cggcgacccg ccgcccgcga     120

gtggccagcc cggcgcagaa ggcccgcgag gagatccagg caatcagcca gaagccggtg     180

gccctgcagg tcgccagtgc gccccacggc agcagcgagg acagcacctc ggcgagcctg     240

ccggcgaact atccctatca cacgcggatg cgccgcaacg agtacgagaa ggccaagcac     300

gacctgcaga tcgaactgct caaggtgcag agctgggtga aggagaccgg ccagcgcgtg     360

gtggtcctgt tcgaaggccg cgacgccgcc ggcaagggcg gcaccatcaa gcgcttcatg     420

gaacacctga acccgcgcgg cgcgcggatc gtagccttgg agaaaccgtc ctcccaggag     480

cagggccagt ggtatttcca gcgctacatc caacatctgc ccaccgccgg cgagatggtc     540

ttcttcgacc gctcctggta caaccgcgcc ggcgtcgagc gggtcatggg cttctgttcg     600

ccgctgcaat acctggagtt catgcgccag gcgccggagc tggagcgcat gctcaccaac     660

agcggcatcc tgctgttcaa gtactggttc tcggtgagcc gcgaggaaca actgcggcgc     720

ttcatctcgc gccgcgacga tccgctcaag cactggaagc tgtcgcccat cgacatcaag     780

tctctggaca agtgggacga ctacaccgcc gccaagcagg cgatgttctt ccataccgac     840

accgccgacg cgccgtggac ggtcatcaag tccgacgaca agaagcgcgc gcgactcaac     900

tgcatccgcc acttcctgca ctcgctggac tacccggaca aggaccggcg catcgcccat     960

gagcccgacc cgttgctggt ggggccggcc tcgcgggtga tcgaggagga cgagaaggtc    1020

tacgccgagg cggccgccgc gccgggccac gcgaacctgg atatcccggc ctga 1074

<210> 12
<211> 903
<212> DNA
<213> Synechococcus sp.

<400> 12
catatggccg agctggacat tactgcggca ccgttagagg cccaaaccga aggtcccggc 60

aagaagaaaa aagcgaaaga taagaagaaa gcgttgccgg aaaccccgaa accgtccaaa 120

ctggatcggg atttctacga caaagaactg gcacgccttc aggtagaact ggtgaaaatg 180

cagtactggg tgaaacacgc tggcttaaaa atcgtgatta tctttgaagg tcgtgatgca 240

gctggcaaag ggggaatgat caaacgcatt tcagcgccgt tgaatccgcg tggatgtcgc 300

attgtggctc tgggtactcc aagtgatcgc gagaaaacgc agtggtactt tcagcgctat 360

gtcgagcatc tacctggtgc aggcgaaata gtcatgttcg atcgtagctg gtacaatagg 420

gcaggtgtgg aatgggtgat gggcttttgc accgaagcgc agtataacga gttcatggat 480

agttgtccac agtttgagcg tatgctggtc aaatccggga taatcctgat taagtactgg 540

tttagcgttt cggatgacga acaagaacgc cgctttcaag ctcgcattct ggaaccggcc 600

aaacgctgga agatctctcc gatggacatc gaatcacggg atcgttgggt tgactattcg 660

aaagccaaag atgccatgct tgcgcatacc aatattccgg aagccccatg gtttacggtt 720

gaagcggacg ataaacgacg tgcgcatctg aactgcatct ctcacttact cagcaaaatc 780

ccgtatgagg acattacacc tcccgccatt gatctcccac cgcgtagacc tgcgcctgaa 840

gattatgtac gtccgccgat taacgaacag ttcttcgttc ccagcatcta ttaataagaa 900

ttc 903

<210> 13
<211> 1068
<212> DNA
<213> Corynebacterium efficiens

<400> 13
catatgaata aaatggaaaa cgctccgatg ccgacgtttg gcaaagaact gcccaaactg 60

gacaacaaag cgtacaaaaa ggagctcaaa cgtctgcaag cagagctggt ggaaatgcag 120

caatgggttg tggaaacggg tactcgcgtc gtgattgtga tggaaggtcg agatgcggcc 180

ggaaaaggct ccgcgattaa gcgcatcacc cagtacctga atccgcgtac agcccgtatt 240

gaggcattac cgactcctac ctctcgcgaa aaaggtcagt ggtatttcca gaggtatgta 300

gagaagttac ctgctgcggg tgagattgtg atctttgatc gcagctggta caatcgcgcc 360

ggcgttgaac gtgttatggg cttttgcacg agtcaggaat atcgccgctt tttgcatcag 420

gcaccgatct tcgaacgctt gttagtcgaa gatggcatac atctccgcaa gtattggttc 480

tctgtatcgg atgaagaaca acttgcccgc tttcatagcc ggctgagtga tccgctgcgt 540

cgttggaaac tgtcgacgat agatctgcac agcattaccc gttgggagga ctactcacgt 600

gcgaaagacg agatgttcat tcacaccgat attcccagcg caccatggta tacagtggaa 660

tcggaggaga aaaaacgcag tcgcatcaac gtaattagcc acatcctttc aaccattccg 720

tacgagaaga ttgaccgtcc gttgccggaa atcccagaac gcccagtgcg ggaaggggag 780

tatatccgtc cacctcggaa cgaatttcgc tatgtcccgg atgttgccgc ttgtctggaa 840

gaacatcgag ttgcagcagc gcgtgaaaaa gcaaaagcag aagctaaagc aagagaagaa 900

gcggaacgtg cgctagccgc ggaaaaagtg aaagcggcca aaaaggcgaa aaagatccgt 960

aaagcccaga aggccaaagc tgcgaaaaaa gccaaaaaag ctgccggtaa ggcgaaagcg 1020

gtcaaaaaaa ccggcaaatc cgggaaaggc ggaaaataat aagaattc 1068

<210> 14
<211> 903
<212> DNA
<213> Kineococcus radiotolerans

<400> 14
catatgccgc atgttcagtt gactccggat ctgggtatga cagttcgcga tgatgaggat 60

gaacctgaac tgcttacgcc agatggcaac gtggtagata cctggcgcga agattacccg 120

tacgacgaac gtctggatcg taaggagtat gatgccgaga aacgtctgct gcaaatcgag 180

ttgctcaaac ttcagcgttg gttgaaagcc tctggtgaac gcatcgtagt gctttgcgaa 240

ggacgcgacg ctgcgggcaa aggcggcacg atcaaacgtt tcatggaaca cctgaatccg 300

cgtggtgccc gggtagttgc actggagaaa ccgtcggaac gtgaaagcac ccagtggtac 360

tttcagcgct atgtccagca cttacccgca gctggcgagt ttgtgctgtt cgatcggagc 420

tggtataatc gcgctggtgt agaacgtgtc atgggttttg cgagtcccgc agaatacgac 480

cgcttcgttg cccaagcccc gttattcgag aaaatgctcg ttgatgatgg gattcacctg 540

gtgaagttct ggttttccgt gactcgcgcg gaacaacgta cccgctttct gattcgccag 600

attgacccgg tgcgccagtg gaaactgtca ccaatggacc tggaaagcct ggaccgctgg 660

gatgaatata ccgcggcgaa agaagcgatg tttgccacca ccgatacgga tgtggcaccg 720

tggacagtcg tcaagaccaa cgacaagaaa cgcgcacgtt tagcggcaat gcgccatgtc 780

ttagcgcgct ttgactatga tgggaaagac ccagaagtgg tgggagttcc ggaccctctg 840

ctggtggttc atgctcggac gattctcgaa gcggatcgtc gccctgcctc gtaatgagaa 900

ttc 903

<210> 15
<211> 1116

<212>  DNA
<213>  Pannonibacter indicus

<400>  15
catatggacg accgtaccgt gacccgcaaa ccacgcggaa cacggccagc accagctgcg      60

gatgccgtgt tgccggtgac cgccgatagc gccgaagctg aagcgacccc tagcggcgtt     120

gctgaaacgc cggcggaggc agtgactgca ccgcaacctg ggacagaacc ggtcgccgct     180

cctgaagccg cgttagaacc ggcgattgcc cctgttgcag cagcaccccg caaaaccctg     240

gctgaaatgc gccatgatcc ggcggcgatt cacgaactgt ttgagtctgg caagtatccg     300

tatgcgaccc cgatgcgtcg cgctccgtat gaacagcgca aagcgaaact ccaagccgaa     360

ctgctgaaag cccaacggtg gatcaaagaa acgggtcaac gcgtggtcat cctgttcgag     420

ggccgtgatg cggcgggcaa aggtgggacc atcaaacgct ttatggagca tctgaatccc     480

cgtggtgcaa ctgtggttgc gttacagaaa cccacggaac aggaagccag ccagtggtac     540

tttcagcgtt acattaacca tctcccgtca ggcggtgaaa tggtcctgtt tgaccgttcg     600

tggtacaatc gggcaggagt tgagcgcgta atgggctttt gcagtgcgtc gcagtatctc     660

gagtttatgc gtcaatgtcc ggaaattgag cgcatgatgg tccgtgatgg gattcgcctg     720

tttaagtact ggttcagcgt gagtcgcgaa gaacagcgtc gccgtttcat ggagcgtcag     780

accgatccgt tgaagcagtg gaagttatca ccaatcgata ttgcctccct ggataaatgg     840

gatgattaca ccgaagcgaa agaagccatg ttcttctata cggacacagc cgacgcgccg     900

tggactatcg tgaaatccga cgataagaaa cgcgcgcgcc tgaactgcat ggagcacttc     960

cttcattctc ttccgtatcc cgacaaagac attcacctgg ttggtgtacc agatcctttg    1020

atcgtaggtg cagctcatca cgtcatttcg cacgatacgc atattctggg caaagccctg    1080

catccggaaa tgaaaccggc aggctaatga gaattc                              1116


<210>  16
<211>  813
<212>  DNA
<213>  Deinococcus radiodurans

<400>  16
catatggata tcgacaacta tcgcgtgaaa cctggtaaac gggtgaaact gtcggattgg      60

gcaacgaacg atgatgcagg cttgtccaaa gaagaaggtc aggcacaaac ggccaaactt     120

gcgggtgaat tggctgaatg gcaggaacgc ctttatgcgg aaggcaagca aagtttactg     180

ctgattctgc aagcacgcga tgcggctggg aaagatggtg ccgttaagaa ggtgattggc     240

gcgtttaacc ctgccggagt tcagatcacc agcttcaaac agccgagtgc ggaagaactg     300

tcacatgact tcttatggcg cattcatcag aaagctcccg cgaaagggta cgtaggcgta     360

tttaaccgtt ctcagtatga agatgtcttg gttactcgcg tctatgacat gatcgatgac     420

aagaccgcta aacgtcgcct ggagcatatt cggcactttg aggaactgct gacggataat 480

gccacacgta tcgtgaaagt ctacctccat attagcccgg aagagcagaa agagcgtctg 540

caagcccgtc ttgacaatcc gggaaaacac tggaaattta acccaggcga cctgaaagac 600

cgctccaatt gggacaaatt caacgatgtc tacgaggatg cgctgactac ctctaccgat 660

gatgcgccgt ggtatgtagt gccagcagat cgcaaatggt atcgtgactt agtgctctcg 720

catattctgc tgggtgccct caaggatatg aatccgcagt ttcccgcgat cgattacgac 780

ccgagcaaag ttgtgattca ctgataagaa ttc 813

<210> 17
<211> 1056
<212> DNA
<213> Gulbenkiania indica

<400> 17
atgaacgaaa aaccgctgat tccggccccg accgatgaaa ccgcggccag cagcgaacag 60

gccgcaccgg ataccccggc ggccgcggcc gcgagcgccc gtagcgcgcg cagccgtcgc 120

cgtcgcccgc cgaccgaaac cagcaaagcc catgatgaaa gcgtgcaggc gattgaagcg 180

gcccagccgg gtccggtggc cctggaagtt gcactggccc cgggcggtag caccgaagat 240

agcaccaccg ccccgctgcc ggcgtgctat ccgtatcgta cccgtatgcg tcgtccggaa 300

tatgaacgcc tgaaagcgga actgcaaatt gaactgctga aagttcagaa ctggattaaa 360

gaaaccggcc agcgcgtgat cgttctgttt gaaggtcgcg atgccgcggg taaaggcggc 420

accatcaaac gttttatgga acatctgaat ccgcgtggcg cccgtgtggt tgcgctggaa 480

aaaccgaccg aagtggaacg cggccagtgg tattttcagc gttatattca gcattttccg 540

accgccggcg aaatcgtgtt tttcgatcgt agctggtata accgcgcggg cgtggaacgt 600

gttatgggtt tttgtacccc gaatgaatat ctggaattta tgcgccaggc cccggaactg 660

gaacgtatgc tggtgaacag cggtattcgc ctgtttaaat tttggtttag cgttagccgt 720

gaagaacagc tgcgtcgctt tatcgcgcgt cgcgatgatc cgctgaaaca ttggaaactg 780

agcccgattg atatccagag cctggataaa tgggatgaat ataccgccgc gaaacagagc 840

atgtttttcc ataccgatac cgccgatgcg ccgtggaccg tgattaaaag cgatgataaa 900

aaacgtgcgc gcattaattg catccgccat tttctgcatc agctgccgta tccggataaa 960

aacccgcgtg ttgcctgtca gccggacccg ctgctggttg gcaatgcgag caaagttctg 1020

gaaccgcatg aaacccaggt tctgaccttt taataa 1056

<210> 18
<211> 948
<212> DNA
<213> Arthrobacter aurescens

<400> 18
atgaccgaag cgagcccgag cagcccgacc ggcaccagcc tggatgattg gtgggttcgt 60

gataacctgc gcgaaaccat tgatcatctg gtggaactgg gctataccat cagcggcggt 120

cagggtgaag atccggatct gattgatccg ggcggtagcg ccgttgaaac ctggaatgaa 180

gattatccgt atcagcagcg tatgacccgc gatgaatacg aaatcgaaaa ataccgcctg 240

caaatcgaac tgctgaaatt tcagtattgg ggccaggatc tgggtctgaa acatgtgatt 300

gtttttgaag gtcgtgatgc ggccggtaaa ggcggcacca tcaaacgttt taccgaacat 360

ctggacccgc gtagcgcccg taccgttgcc ctggcaaaac cgagcgatcg cgaacagggc 420

cagtggtatt ttcagcgtta tattcagcag tttccgaccg cgggtgaaat cgtgatgttt 480

gatcgtagct ggtataaccg cgccaatgtg gaacgtgtta tgggcttttg caccgatgat 540

gaatatgata cctttatggg tcaggcgccg gtgtttgaaa aaatgctggt tgatgccggt 600

atccatgtga ccaaattttg gtttagcgtt acccgccagg aacagcgtac ccgctttgcg 660

attcgtcaga tcgatccggt gcgtcgctgg aaactgagcc cgatggatct ggcgagcctg 720

gatcgctggg atgaatatac cgatgccaaa gaacgtacct ttctgcatac cgatagcgat 780

catgccccgt ggattaccat caaaagcaac gataaaaaac gtgcgcgcat taacgccatg 840

cgctattttc tgaaccagtt cgattacgaa gataaagata ccagcgtggt ttatgatgcc 900

gatccgctga ttctgcgtcg cggtcgtgat gcagtgggtg attaataa 948

<210> 19
<211> 813
<212> DNA
<213> Thiobacillus denitrificans

<400> 19
atgaaaccgc gtgatacccg cgtgaaaccg ggcagccgtg ttagcctggc ggattggcat 60

accgatggcg atgcctttgt gggtaaagat aaagcggcca gcatggcgcg tctggatgat 120

gatcgtgttc gcctggaaga actgcaagaa ctgctgtatg ccgaaggcaa acatcgcctg 180

ctggttgtgc tgcaagcgat ggataccgcc ggtaaagata gcaccattcg tcatgtgttt 240

cgcggcgttg atccgctggg tgtgcgcgtt gcgaactttg gcgtgccgag cacccatgaa 300

ctgcgtcatg attatctgtg gcgtgtgcat ccgcatgttc cggccagcgg tgaaattgcc 360

atctttaacc gtagccatta tgaagatgtg ctggttccgc gcgttaatgg cgcgattggt 420

catgccgaat gcgaacgtcg ctatcgtcag atcaatgatt ttgaacgtat gctgagcgaa 480

accggcacca ccatccgtaa attctatctg catatcagca aagatgaaca gaaaaaacgt 540

ctggaagcgc gtcgcgatac cccgaaaaaa cgctggaaat ttcagccggg tgatctggcc 600

gtgcgtgcac agtgggatga ttatcgtgcc gcctatgatg ccctgctgag cgcaaccagc 660

acccgtcatg ccccgtggca tgtggttccg gcggatgata aactggcccg caacctgatt 720

gtgagcgcgc tgctgatcga agccctggaa ggtctggata tgcgctatcc ggaaccggtg 780

gcgggcgttg cgggcacccc gattatctaa taa 813

<210> 20
<211> 1092
<212> DNA
<213> Pseudomonas fluorescens

<400> 20
atgagcgaag aaagcaccgc cctgccgctg ccgccggcac cggtgcagaa agcgccggcc 60

agcaccagcc cggcgagcac caaagcggcg ccgcgtaaag ccgcaacccc gcgtccgcgt 120

cgcccgcgta ccaccaaagc ggccccggtt aaagcggccg aagcggaaat tagcgccatc 180

agccagaaac cgatggccct gcaagtggcc aatgccccgc gtggcagcaa tgaagatagc 240

gttagcgcgg ccctgccggg taactatccg tatcgtaatc gtatgcgtcg tgcggaatat 300

gaaaaagcca aaaacgaact gcaaattgaa ctgctgaaag tgcagagctg ggttaaagaa 360

accggccagc gcatcgtggt tctgtttgaa ggtcgcgatg cggccggtaa aggcggcacc 420

attaaacgct ttatggaaca tctgaatccg cgtggcgcgc gtattgtggc cctggaaaaa 480

ccgagcgaac aggaaaaagg ccagtggtat tttcagcgtt atattcagca tctgccgacc 540

gcgggcgaaa tggtgttttt cgatcgtagc tggtataacc gcgccggtgt ggaacgtgtt 600

atggaatttt gcagcccgct gcaatatctg gaatttatgc gccagacccc ggaactggaa 660

cgtatgctgt gtaacagcgg tattctgatg ttcaaattct ggttcagcgt gaatcgcgaa 720

gaacagctgc gtcgctttat cagccgtcgc gatgatccgc tgaaacattg gaaactgagc 780

ccgattgata tcaaaagcct ggataaatgg gatgaatata ccgcggccaa acaggcgatg 840

tttttccata ccgataccgc ggatgccccg tggaccgtta tcaaaagcga tgataaaaaa 900

cgtgcgcgca ttaattgcat ccgccatttt ctgcatgaac tggattatcc gggcaaagat 960

ctgaaagtgg cccatgcgcc ggacccgctg ctggttggcc gtgccagccg cggtctggaa 1020

gaagatgaac gtacccaggc acaggccgca accgatgccg gtgcaaccaa actggccctg 1080

agcgcataat aa 1092

SEQUENCE LISTING

<110>  KANEKA CORPORATION

<120>  METHOD OF PRODUCING SUBSTANCE USING ATP

<130>  B170312

<150>  JP 2017-091451
<151>  2017-05-01

<160>  20

<170>  PatentIn version 3.5

<210>  1
<211>  357
<212>  PRT
<213>  Pseudomonas aeruginosa

<400>  1

Met Ser Glu Glu Pro Thr Val Ser Pro Pro Ser Pro Glu Gln Pro Ala
1               5                   10                  15

Ala Gln Pro Ala Lys Pro Ala Arg Pro Ala Ala Arg Arg Ala Pro Arg
            20                  25                  30

Lys Pro Ala Thr Arg Arg Pro Arg Val Ala Ser Pro Ala Gln Lys Ala
        35                  40                  45

Arg Glu Glu Ile Gln Ala Ile Ser Gln Lys Pro Val Ala Leu Gln Val
    50                  55                  60

Ala Ser Ala Pro His Gly Ser Ser Glu Asp Ser Thr Ser Ala Ser Leu
65                  70                  75                  80

Pro Ala Asn Tyr Pro Tyr His Thr Arg Met Arg Arg Asn Glu Tyr Glu
                85                  90                  95

Lys Ala Lys His Asp Leu Gln Ile Glu Leu Leu Lys Val Gln Ser Trp
            100                 105                 110

Val Lys Glu Thr Gly Gln Arg Val Val Val Leu Phe Glu Gly Arg Asp
        115                 120                 125

Ala Ala Gly Lys Gly Gly Thr Ile Lys Arg Phe Met Glu His Leu Asn
    130                 135                 140

Pro Arg Gly Ala Arg Ile Val Ala Leu Glu Lys Pro Ser Ser Gln Glu
145                 150                 155                 160

Gln Gly Gln Trp Tyr Phe Gln Arg Tyr Ile Gln His Leu Pro Thr Ala

165                 170                 175

Gly Glu Met Val Phe Phe Asp Arg Ser Trp Tyr Asn Arg Ala Gly Val
            180                 185                 190

Glu Arg Val Met Gly Phe Cys Ser Pro Leu Gln Tyr Leu Glu Phe Met
        195                 200                 205

Arg Gln Ala Pro Glu Leu Glu Arg Met Leu Thr Asn Ser Gly Ile Leu
    210                 215                 220

Leu Phe Lys Tyr Trp Phe Ser Val Ser Arg Glu Glu Gln Leu Arg Arg
225                 230                 235                 240

Phe Ile Ser Arg Arg Asp Asp Pro Leu Lys His Trp Lys Leu Ser Pro
                245                 250                 255

Ile Asp Ile Lys Ser Leu Asp Lys Trp Asp Asp Tyr Thr Ala Ala Lys
            260                 265                 270

Gln Ala Met Phe Phe His Thr Asp Thr Ala Asp Ala Pro Trp Thr Val
        275                 280                 285

Ile Lys Ser Asp Asp Lys Lys Arg Ala Arg Leu Asn Cys Ile Arg His
    290                 295                 300

Phe Leu His Ser Leu Asp Tyr Pro Asp Lys Asp Arg Arg Ile Ala His
305                 310                 315                 320

Glu Pro Asp Pro Leu Leu Val Gly Pro Ala Ser Arg Val Ile Glu Glu
                325                 330                 335

Asp Glu Lys Val Tyr Ala Glu Ala Ala Ala Ala Pro Gly His Ala Asn
            340                 345                 350

Leu Asp Ile Pro Ala
        355

<210>  2
<211>  296
<212>  PRT
<213>  Synechococcus sp.

<400>  2

Met Ala Glu Leu Asp Ile Thr Ala Ala Pro Leu Glu Ala Gln Thr Glu
1               5                   10                  15

Gly Pro Gly Lys Lys Lys Lys Ala Lys Asp Lys Lys Lys Ala Leu Pro

20                  25                  30

Glu Thr Pro Lys Pro Ser Lys Leu Asp Arg Asp Phe Tyr Asp Lys Glu
        35                  40                  45

Leu Ala Arg Leu Gln Val Glu Leu Val Lys Met Gln Tyr Trp Val Lys
    50                  55                  60

His Ala Gly Leu Lys Ile Val Ile Ile Phe Glu Gly Arg Asp Ala Ala
65                  70                  75                  80

Gly Lys Gly Gly Met Ile Lys Arg Ile Ser Ala Pro Leu Asn Pro Arg
                85                  90                  95

Gly Cys Arg Ile Val Ala Leu Gly Thr Pro Ser Asp Arg Glu Lys Thr
            100                 105                 110

Gln Trp Tyr Phe Gln Arg Tyr Val Glu His Leu Pro Gly Ala Gly Glu
        115                 120                 125

Ile Val Met Phe Asp Arg Ser Trp Tyr Asn Arg Ala Gly Val Glu Trp
    130                 135                 140

Val Met Gly Phe Cys Thr Glu Ala Gln Tyr Asn Glu Phe Met Asp Ser
145                 150                 155                 160

Cys Pro Gln Phe Glu Arg Met Leu Val Lys Ser Gly Ile Ile Leu Ile
                165                 170                 175

Lys Tyr Trp Phe Ser Val Ser Asp Asp Glu Gln Glu Arg Arg Phe Gln
            180                 185                 190

Ala Arg Ile Leu Glu Pro Ala Lys Arg Trp Lys Ile Ser Pro Met Asp
        195                 200                 205

Ile Glu Ser Arg Asp Arg Trp Val Asp Tyr Ser Lys Ala Lys Asp Ala
    210                 215                 220

Met Leu Ala His Thr Asn Ile Pro Glu Ala Pro Trp Phe Thr Val Glu
225                 230                 235                 240

Ala Asp Asp Lys Arg Arg Ala His Leu Asn Cys Ile Ser His Leu Leu
                245                 250                 255

Ser Lys Ile Pro Tyr Glu Asp Ile Thr Pro Pro Ala Ile Asp Leu Pro
            260                 265                 270

Pro Arg Arg Pro Ala Pro Glu Asp Tyr Val Arg Pro Pro Ile Asn Glu
        275                 280                 285

Gln Phe Phe Val Pro Ser Ile Tyr
    290                 295

<210>  3
<211>  351
<212>  PRT
<213>  Corynebacterium efficiens

<400>  3

Met Asn Lys Met Glu Asn Ala Pro Met Pro Thr Phe Gly Lys Glu Leu
1               5                   10                  15

Pro Lys Leu Asp Asn Lys Ala Tyr Lys Lys Glu Leu Lys Arg Leu Gln
            20                  25                  30

Ala Glu Leu Val Glu Met Gln Gln Trp Val Val Glu Thr Gly Thr Arg
        35                  40                  45

Val Val Ile Val Met Glu Gly Arg Asp Ala Ala Gly Lys Gly Ser Ala
    50                  55                  60

Ile Lys Arg Ile Thr Gln Tyr Leu Asn Pro Arg Thr Ala Arg Ile Glu
65                  70                  75                  80

Ala Leu Pro Thr Pro Thr Ser Arg Glu Lys Gly Gln Trp Tyr Phe Gln
                85                  90                  95

Arg Tyr Val Glu Lys Leu Pro Ala Ala Gly Glu Ile Val Ile Phe Asp
            100                 105                 110

Arg Ser Trp Tyr Asn Arg Ala Gly Val Glu Arg Val Met Gly Phe Cys
        115                 120                 125

Thr Ser Gln Glu Tyr Arg Arg Phe Leu His Gln Ala Pro Ile Phe Glu
    130                 135                 140

Arg Leu Leu Val Glu Asp Gly Ile His Leu Arg Lys Tyr Trp Phe Ser
145                 150                 155                 160

Val Ser Asp Glu Glu Gln Leu Ala Arg Phe His Ser Arg Leu Ser Asp
                165                 170                 175

Pro Leu Arg Arg Trp Lys Leu Ser Thr Ile Asp Leu His Ser Ile Thr
            180                 185                 190

Arg Trp Glu Asp Tyr Ser Arg Ala Lys Asp Glu Met Phe Ile His Thr
        195                 200                 205

Asp Ile Pro Ser Ala Pro Trp Tyr Thr Val Glu Ser Glu Glu Lys Lys
    210                 215                 220

Arg Ser Arg Ile Asn Val Ile Ser His Ile Leu Ser Thr Ile Pro Tyr
225                 230                 235                 240

Glu Lys Ile Asp Arg Pro Leu Pro Glu Ile Pro Glu Arg Pro Val Arg
                245                 250                 255

Glu Gly Glu Tyr Ile Arg Pro Pro Arg Asn Glu Phe Arg Tyr Val Pro
            260                 265                 270

Asp Val Ala Ala Cys Leu Glu Glu His Arg Val Ala Ala Ala Arg Glu
        275                 280                 285

Lys Ala Lys Ala Glu Ala Lys Ala Arg Glu Glu Ala Glu Arg Ala Leu
    290                 295                 300

Ala Ala Glu Lys Val Lys Ala Ala Lys Lys Ala Lys Lys Ile Arg Lys
305                 310                 315                 320

Ala Gln Lys Ala Lys Ala Ala Lys Lys Ala Lys Lys Ala Ala Gly Lys
                325                 330                 335

Ala Lys Ala Val Lys Lys Thr Gly Lys Ser Gly Lys Gly Gly Lys
            340                 345                 350

<210>  4
<211>  296
<212>  PRT
<213>  Kineococcus radiotolerans

<400>  4

Met Pro His Val Gln Leu Thr Pro Asp Leu Gly Met Thr Val Arg Asp
1               5                   10                  15

Asp Glu Asp Glu Pro Glu Leu Leu Thr Pro Asp Gly Asn Val Val Asp
            20                  25                  30

Thr Trp Arg Glu Asp Tyr Pro Tyr Asp Glu Arg Leu Asp Arg Lys Glu
        35                  40                  45

Tyr Asp Ala Glu Lys Arg Leu Leu Gln Ile Glu Leu Leu Lys Leu Gln
    50                  55                  60

Arg Trp Leu Lys Ala Ser Gly Glu Arg Ile Val Val Leu Cys Glu Gly
65                  70                  75                  80

Arg Asp Ala Ala Gly Lys Gly Gly Thr Ile Lys Arg Phe Met Glu His
                85                  90                  95

Leu Asn Pro Arg Gly Ala Arg Val Val Ala Leu Glu Lys Pro Ser Glu
            100                 105                 110

Arg Glu Ser Thr Gln Trp Tyr Phe Gln Arg Tyr Val Gln His Leu Pro
        115                 120                 125

Ala Ala Gly Glu Phe Val Leu Phe Asp Arg Ser Trp Tyr Asn Arg Ala
    130                 135                 140

Gly Val Glu Arg Val Met Gly Phe Ala Ser Pro Ala Glu Tyr Asp Arg
145                 150                 155                 160

Phe Val Ala Gln Ala Pro Leu Phe Glu Lys Met Leu Val Asp Asp Gly
                165                 170                 175

Ile His Leu Val Lys Phe Trp Phe Ser Val Thr Arg Ala Glu Gln Arg
            180                 185                 190

Thr Arg Phe Leu Ile Arg Gln Ile Asp Pro Val Arg Gln Trp Lys Leu
        195                 200                 205

Ser Pro Met Asp Leu Glu Ser Leu Asp Arg Trp Asp Glu Tyr Thr Ala
    210                 215                 220

Ala Lys Glu Ala Met Phe Ala Thr Thr Asp Thr Asp Val Ala Pro Trp
225                 230                 235                 240

Thr Val Val Lys Thr Asn Asp Lys Lys Arg Ala Arg Leu Ala Ala Met
                245                 250                 255

Arg His Val Leu Ala Arg Phe Asp Tyr Asp Gly Lys Asp Pro Glu Val
            260                 265                 270

Val Gly Val Pro Asp Pro Leu Leu Val Val His Ala Arg Thr Ile Leu
        275                 280                 285

Glu Ala Asp Arg Arg Pro Ala Ser
    290                 295

<210>  5
<211>  367
<212>  PRT

<213>  Pannonibacter indicus

<400>  5

Met Asp Asp Arg Thr Val Thr Arg Lys Pro Arg Gly Thr Arg Pro Ala
1               5                   10                  15

Pro Ala Ala Asp Ala Val Leu Pro Val Thr Ala Asp Ser Ala Glu Ala
            20                  25                  30

Glu Ala Thr Pro Ser Gly Val Ala Glu Thr Pro Ala Glu Ala Val Thr
        35                  40                  45

Ala Pro Gln Pro Gly Thr Glu Pro Val Ala Ala Pro Glu Ala Ala Leu
    50                  55                  60

Glu Pro Ala Ile Ala Pro Val Ala Ala Ala Pro Arg Lys Thr Leu Ala
65                  70                  75                  80

Glu Met Arg His Asp Pro Ala Ala Ile His Glu Leu Phe Glu Ser Gly
                85                  90                  95

Lys Tyr Pro Tyr Ala Thr Pro Met Arg Arg Ala Pro Tyr Glu Gln Arg
            100                 105                 110

Lys Ala Lys Leu Gln Ala Glu Leu Leu Lys Ala Gln Arg Trp Ile Lys
        115                 120                 125

Glu Thr Gly Gln Arg Val Val Ile Leu Phe Glu Gly Arg Asp Ala Ala
    130                 135                 140

Gly Lys Gly Gly Thr Ile Lys Arg Phe Met Glu His Leu Asn Pro Arg
145                 150                 155                 160

Gly Ala Thr Val Val Ala Leu Gln Lys Pro Thr Glu Gln Glu Ala Ser
                165                 170                 175

Gln Trp Tyr Phe Gln Arg Tyr Ile Asn His Leu Pro Ser Gly Gly Glu
            180                 185                 190

Met Val Leu Phe Asp Arg Ser Trp Tyr Asn Arg Ala Gly Val Glu Arg
        195                 200                 205

Val Met Gly Phe Cys Ser Ala Ser Gln Tyr Leu Glu Phe Met Arg Gln
    210                 215                 220

Cys Pro Glu Ile Glu Arg Met Met Val Arg Asp Gly Ile Arg Leu Phe
225                 230                 235                 240

Lys Tyr Trp Phe Ser Val Ser Arg Glu Glu Gln Arg Arg Arg Phe Met
                245                 250                 255

Glu Arg Gln Thr Asp Pro Leu Lys Gln Trp Lys Leu Ser Pro Ile Asp
            260                 265                 270

Ile Ala Ser Leu Asp Lys Trp Asp Asp Tyr Thr Glu Ala Lys Glu Ala
        275                 280                 285

Met Phe Phe Tyr Thr Asp Thr Ala Asp Ala Pro Trp Thr Ile Val Lys
    290                 295                 300

Ser Asp Asp Lys Lys Arg Ala Arg Leu Asn Cys Met Glu His Phe Leu
305                 310                 315                 320

His Ser Leu Pro Tyr Pro Asp Lys Asp Ile His Leu Val Gly Val Pro
                325                 330                 335

Asp Pro Leu Ile Val Gly Ala Ala His His Val Ile Ser His Asp Thr
            340                 345                 350

His Ile Leu Gly Lys Ala Leu His Pro Glu Met Lys Pro Ala Gly
        355                 360                 365

<210>  6
<211>  266
<212>  PRT
<213>  Deinococcus radiodurans

<400>  6

Met Asp Ile Asp Asn Tyr Arg Val Lys Pro Gly Lys Arg Val Lys Leu
1               5                   10                  15

Ser Asp Trp Ala Thr Asn Asp Asp Ala Gly Leu Ser Lys Glu Glu Gly
            20                  25                  30

Gln Ala Gln Thr Ala Lys Leu Ala Gly Glu Leu Ala Glu Trp Gln Glu
        35                  40                  45

Arg Leu Tyr Ala Glu Gly Lys Gln Ser Leu Leu Leu Ile Leu Gln Ala
    50                  55                  60

Arg Asp Ala Ala Gly Lys Asp Gly Ala Val Lys Lys Val Ile Gly Ala
65                  70                  75                  80

Phe Asn Pro Ala Gly Val Gln Ile Thr Ser Phe Lys Gln Pro Ser Ala
                85                  90                  95

Glu Glu Leu Ser His Asp Phe Leu Trp Arg Ile His Gln Lys Ala Pro
            100                 105                 110

Ala Lys Gly Tyr Val Gly Val Phe Asn Arg Ser Gln Tyr Glu Asp Val
        115                 120                 125

Leu Val Thr Arg Val Tyr Asp Met Ile Asp Asp Lys Thr Ala Lys Arg
    130                 135                 140

Arg Leu Glu His Ile Arg His Phe Glu Glu Leu Leu Thr Asp Asn Ala
145                 150                 155                 160

Thr Arg Ile Val Lys Val Tyr Leu His Ile Ser Pro Glu Glu Gln Lys
                165                 170                 175

Glu Arg Leu Gln Ala Arg Leu Asp Asn Pro Gly Lys His Trp Lys Phe
            180                 185                 190

Asn Pro Gly Asp Leu Lys Asp Arg Ser Asn Trp Asp Lys Phe Asn Asp
        195                 200                 205

Val Tyr Glu Asp Ala Leu Thr Thr Ser Thr Asp Asp Ala Pro Trp Tyr
    210                 215                 220

Val Val Pro Ala Asp Arg Lys Trp Tyr Arg Asp Leu Val Leu Ser His
225                 230                 235                 240

Ile Leu Leu Gly Ala Leu Lys Asp Met Asn Pro Gln Phe Pro Ala Ile
                245                 250                 255

Asp Tyr Asp Pro Ser Lys Val Val Ile His
            260                 265

<210>  7
<211>  350
<212>  PRT
<213>  Gulbenkiania indica

<400>  7

Met Asn Glu Lys Pro Leu Ile Pro Ala Pro Thr Asp Glu Thr Ala Ala
1               5                   10                  15

Ser Ser Glu Gln Ala Ala Pro Asp Thr Pro Ala Ala Ala Ala Ala Ser
            20                  25                  30

Ala Arg Ser Ala Arg Ser Arg Arg Arg Pro Pro Thr Glu Thr Ser
        35                  40                  45

Lys Ala His Asp Glu Ser Val Gln Ala Ile Glu Ala Ala Gln Pro Gly
    50                  55                  60

Pro Val Ala Leu Glu Val Ala Leu Ala Pro Gly Gly Ser Thr Glu Asp
65                  70                  75                  80

Ser Thr Thr Ala Pro Leu Pro Ala Cys Tyr Pro Tyr Arg Thr Arg Met
                85                  90                  95

Arg Arg Pro Glu Tyr Glu Arg Leu Lys Ala Glu Leu Gln Ile Glu Leu
            100                 105                 110

Leu Lys Val Gln Asn Trp Ile Lys Glu Thr Gly Gln Arg Val Ile Val
        115                 120                 125

Leu Phe Glu Gly Arg Asp Ala Ala Gly Lys Gly Gly Thr Ile Lys Arg
    130                 135                 140

Phe Met Glu His Leu Asn Pro Arg Gly Ala Arg Val Val Ala Leu Glu
145                 150                 155                 160

Lys Pro Thr Glu Val Glu Arg Gly Gln Trp Tyr Phe Gln Arg Tyr Ile
                165                 170                 175

Gln His Phe Pro Thr Ala Gly Glu Ile Val Phe Phe Asp Arg Ser Trp
            180                 185                 190

Tyr Asn Arg Ala Gly Val Glu Arg Val Met Gly Phe Cys Thr Pro Asn
        195                 200                 205

Glu Tyr Leu Glu Phe Met Arg Gln Ala Pro Glu Leu Glu Arg Met Leu
    210                 215                 220

Val Asn Ser Gly Ile Arg Leu Phe Lys Phe Trp Phe Ser Val Ser Arg
225                 230                 235                 240

Glu Glu Gln Leu Arg Arg Phe Ile Ala Arg Arg Asp Asp Pro Leu Lys
                245                 250                 255

His Trp Lys Leu Ser Pro Ile Asp Ile Gln Ser Leu Asp Lys Trp Asp
            260                 265                 270

Glu Tyr Thr Ala Ala Lys Gln Ser Met Phe Phe His Thr Asp Thr Ala
        275                 280                 285

Asp Ala Pro Trp Thr Val Ile Lys Ser Asp Asp Lys Lys Arg Ala Arg
    290                 295                 300

Ile Asn Cys Ile Arg His Phe Leu His Gln Leu Pro Tyr Pro Asp Lys
305                 310                 315                 320

Asn Pro Arg Val Ala Cys Gln Pro Asp Pro Leu Leu Val Gly Asn Ala
                325                 330                 335

Ser Lys Val Leu Glu Pro His Glu Thr Gln Val Leu Thr Phe
            340                 345                 350

<210>  8
<211>  314
<212>  PRT
<213>  Arthrobacter aurescens

<400>  8

Met Thr Glu Ala Ser Pro Ser Ser Pro Thr Gly Thr Ser Leu Asp Asp
1               5                   10                  15

Trp Trp Val Arg Asp Asn Leu Arg Glu Thr Ile Asp His Leu Val Glu
            20                  25                  30

Leu Gly Tyr Thr Ile Ser Gly Gly Gln Gly Glu Asp Pro Asp Leu Ile
        35                  40                  45

Asp Pro Gly Gly Ser Ala Val Glu Thr Trp Asn Glu Asp Tyr Pro Tyr
    50                  55                  60

Gln Gln Arg Met Thr Arg Asp Glu Tyr Glu Ile Glu Lys Tyr Arg Leu
65                  70                  75                  80

Gln Ile Glu Leu Leu Lys Phe Gln Tyr Trp Gly Gln Asp Leu Gly Leu
                85                  90                  95

Lys His Val Ile Val Phe Glu Gly Arg Asp Ala Ala Gly Lys Gly Gly
            100                 105                 110

Thr Ile Lys Arg Phe Thr Glu His Leu Asp Pro Arg Ser Ala Arg Thr
        115                 120                 125

Val Ala Leu Ala Lys Pro Ser Asp Arg Glu Gln Gly Gln Trp Tyr Phe
    130                 135                 140

Gln Arg Tyr Ile Gln Gln Phe Pro Thr Ala Gly Glu Ile Val Met Phe
145                 150                 155                 160

Asp Arg Ser Trp Tyr Asn Arg Ala Asn Val Glu Arg Val Met Gly Phe
                165                 170                 175

Cys Thr Asp Asp Glu Tyr Asp Thr Phe Met Gly Gln Ala Pro Val Phe
            180                 185                 190

Glu Lys Met Leu Val Asp Ala Gly Ile His Val Thr Lys Phe Trp Phe
        195                 200                 205

Ser Val Thr Arg Gln Glu Gln Arg Thr Arg Phe Ala Ile Arg Gln Ile
    210                 215                 220

Asp Pro Val Arg Arg Trp Lys Leu Ser Pro Met Asp Leu Ala Ser Leu
225                 230                 235                 240

Asp Arg Trp Asp Glu Tyr Thr Asp Ala Lys Glu Arg Thr Phe Leu His
                245                 250                 255

Thr Asp Ser Asp His Ala Pro Trp Ile Thr Ile Lys Ser Asn Asp Lys
            260                 265                 270

Lys Arg Ala Arg Ile Asn Ala Met Arg Tyr Phe Leu Asn Gln Phe Asp
        275                 280                 285

Tyr Glu Asp Lys Asp Thr Ser Val Val Tyr Asp Ala Asp Pro Leu Ile
    290                 295                 300

Leu Arg Arg Gly Arg Asp Ala Val Gly Asp
305                 310

<210>  9
<211>  269
<212>  PRT
<213>  Thiobacillus denitrificans

<400>  9

Met Lys Pro Arg Asp Thr Arg Val Lys Pro Gly Ser Arg Val Ser Leu
1               5                   10                  15

Ala Asp Trp His Thr Asp Gly Asp Ala Phe Val Gly Lys Asp Lys Ala
            20                  25                  30

Ala Ser Met Ala Arg Leu Asp Asp Asp Arg Val Arg Leu Glu Glu Leu
        35                  40                  45

Gln Glu Leu Leu Tyr Ala Glu Gly Lys His Arg Leu Leu Val Val Leu
    50                  55                  60

Gln Ala Met Asp Thr Ala Gly Lys Asp Ser Thr Ile Arg His Val Phe
65                  70                  75                  80

Arg Gly Val Asp Pro Leu Gly Val Arg Val Ala Asn Phe Gly Val Pro
                85                  90                  95

Ser Thr His Glu Leu Arg His Asp Tyr Leu Trp Arg Val His Pro His
            100                 105                 110

Val Pro Ala Ser Gly Glu Ile Ala Ile Phe Asn Arg Ser His Tyr Glu
        115                 120                 125

Asp Val Leu Val Pro Arg Val Asn Gly Ala Ile Gly His Ala Glu Cys
    130                 135                 140

Glu Arg Arg Tyr Arg Gln Ile Asn Asp Phe Glu Arg Met Leu Ser Glu
145                 150                 155                 160

Thr Gly Thr Thr Ile Arg Lys Phe Tyr Leu His Ile Ser Lys Asp Glu
                165                 170                 175

Gln Lys Lys Arg Leu Glu Ala Arg Arg Asp Thr Pro Lys Lys Arg Trp
            180                 185                 190

Lys Phe Gln Pro Gly Asp Leu Ala Val Arg Ala Gln Trp Asp Asp Tyr
        195                 200                 205

Arg Ala Ala Tyr Asp Ala Leu Leu Ser Ala Thr Ser Thr Arg His Ala
    210                 215                 220

Pro Trp His Val Val Pro Ala Asp Asp Lys Leu Ala Arg Asn Leu Ile
225                 230                 235                 240

Val Ser Ala Leu Leu Ile Glu Ala Leu Glu Gly Leu Asp Met Arg Tyr
                245                 250                 255

Pro Glu Pro Val Ala Gly Val Ala Gly Thr Pro Ile Ile
            260                 265

<210>  10
<211>  362
<212>  PRT
<213>  Pseudomonas fluorescens

<400>  10

Met Ser Glu Glu Ser Thr Ala Leu Pro Leu Pro Pro Ala Pro Val Gln
1               5                   10                  15

Lys Ala Pro Ala Ser Thr Ser Pro Ala Ser Thr Lys Ala Ala Pro Arg
            20                  25                  30

Lys Ala Ala Thr Pro Arg Pro Arg Arg Pro Arg Thr Thr Lys Ala Ala
        35                  40                  45

Pro Val Lys Ala Ala Glu Ala Glu Ile Ser Ala Ile Ser Gln Lys Pro
    50                  55                  60

Met Ala Leu Gln Val Ala Asn Ala Pro Arg Gly Ser Asn Glu Asp Ser
65                  70                  75                  80

Val Ser Ala Ala Leu Pro Gly Asn Tyr Pro Tyr Arg Asn Arg Met Arg
                85                  90                  95

Arg Ala Glu Tyr Glu Lys Ala Lys Asn Glu Leu Gln Ile Glu Leu Leu
            100                 105                 110

Lys Val Gln Ser Trp Val Lys Glu Thr Gly Gln Arg Ile Val Val Leu
        115                 120                 125

Phe Glu Gly Arg Asp Ala Ala Gly Lys Gly Gly Thr Ile Lys Arg Phe
    130                 135                 140

Met Glu His Leu Asn Pro Arg Gly Ala Arg Ile Val Ala Leu Glu Lys
145                 150                 155                 160

Pro Ser Glu Gln Glu Lys Gly Gln Trp Tyr Phe Gln Arg Tyr Ile Gln
                165                 170                 175

His Leu Pro Thr Ala Gly Glu Met Val Phe Phe Asp Arg Ser Trp Tyr
            180                 185                 190

Asn Arg Ala Gly Val Glu Arg Val Met Glu Phe Cys Ser Pro Leu Gln
        195                 200                 205

Tyr Leu Glu Phe Met Arg Gln Thr Pro Glu Leu Glu Arg Met Leu Cys
    210                 215                 220

Asn Ser Gly Ile Leu Met Phe Lys Phe Trp Phe Ser Val Asn Arg Glu
225                 230                 235                 240

Glu Gln Leu Arg Arg Phe Ile Ser Arg Arg Asp Asp Pro Leu Lys His
                245                 250                 255

Trp Lys Leu Ser Pro Ile Asp Ile Lys Ser Leu Asp Lys Trp Asp Glu
            260                 265                 270

Tyr Thr Ala Ala Lys Gln Ala Met Phe Phe His Thr Asp Thr Ala Asp

275                 280                 285

Ala Pro Trp Thr Val Ile Lys Ser Asp Asp Lys Lys Arg Ala Arg Ile
    290                 295                 300

Asn Cys Ile Arg His Phe Leu His Glu Leu Asp Tyr Pro Gly Lys Asp
305                 310                 315                 320

Leu Lys Val Ala His Ala Pro Asp Pro Leu Leu Val Gly Arg Ala Ser
                325                 330                 335

Arg Gly Leu Glu Glu Asp Glu Arg Thr Gln Ala Gln Ala Ala Thr Asp
            340                 345                 350

Ala Gly Ala Thr Lys Leu Ala Leu Ser Ala
        355                 360

<210>  11
<211>  1074
<212>  DNA
<213>  Pseudomonas aeruginosa

<400>  11
atgagcgaag aacccactgt cagtcccccc tcccccgagc aacccgccgc gcagccggcc      60

aagccggccc ggccagccgc ccgccgcgcc ccgcgcaagc cggcgacccg ccgcccgcga     120

gtggccagcc cggcgcagaa ggcccgcgag gagatccagg caatcagcca gaagccggtg     180

gccctgcagg tcgccagtgc gccccacggc agcagcgagg acagcacctc ggcgagcctg     240

ccggcgaact atccctatca cacgcggatg cgccgcaacg agtacgagaa ggccaagcac     300

gacctgcaga tcgaactgct caaggtgcag agctgggtga aggagaccgg ccagcgcgtg     360

gtggtcctgt tcgaaggccg cgacgccgcc ggcaagggcg gcaccatcaa gcgcttcatg     420

gaacacctga acccgcgcgg cgcgcggatc gtagccttgg agaaaccgtc ctcccaggag     480

cagggccagt ggtatttcca gcgctacatc caacatctgc ccaccgccgg cgagatggtc     540

ttcttcgacc gctcctggta caaccgcgcc ggcgtcgagc gggtcatggg cttctgttcg     600

ccgctgcaat acctggagtt catgcgccag gcgccggagc tggagcgcat gctcaccaac     660

agcggcatcc tgctgttcaa gtactggttc tcggtgagcc gcgaggaaca actgcggcgc     720

ttcatctcgc gccgcgacga tccgctcaag cactggaagc tgtcgcccat cgacatcaag     780

tctctggaca agtgggacga ctacaccgcc gccaagcagg cgatgttctt ccataccgac     840

accgccgacg cgccgtggac ggtcatcaag tccgacgaca agaagcgcgc gcgactcaac     900

tgcatccgcc acttcctgca ctcgctggac tacccggaca aggaccggcg catcgcccat     960

gagcccgacc cgttgctggt ggggccggcc tcgcgggtga tcgaggagga cgagaaggtc    1020

tacgccgagg cggccgccgc gccgggccac gcgaacctgg atatcccggc ctga 1074

<210> 12
<211> 903
<212> DNA
<213> Synechococcus sp.

<400> 12
catatggccg agctggacat tactgcggca ccgttagagg cccaaaccga aggtcccggc 60

aagaagaaaa aagcgaaaga taagaagaaa gcgttgccgg aaaccccgaa accgtccaaa 120

ctggatcggg atttctacga caaagaactg gcacgccttc aggtagaact ggtgaaaatg 180

cagtactggg tgaaacacgc tggcttaaaa atcgtgatta tctttgaagg tcgtgatgca 240

gctggcaaag ggggaatgat caaacgcatt tcagcgccgt tgaatccgcg tggatgtcgc 300

attgtggctc tgggtactcc aagtgatcgc gagaaaacgc agtggtactt tcagcgctat 360

gtcgagcatc tacctggtgc aggcgaaata gtcatgttcg atcgtagctg gtacaatagg 420

gcaggtgtgg aatgggtgat gggcttttgc accgaagcgc agtataacga gttcatggat 480

agttgtccac agtttgagcg tatgctggtc aaatccggga taatcctgat taagtactgg 540

tttagcgttt cggatgacga acaagaacgc cgctttcaag ctcgcattct ggaaccggcc 600

aaacgctgga agatctctcc gatggacatc gaatcacggg atcgttgggt tgactattcg 660

aaagccaaag atgccatgct tgcgcatacc aatattccgg aagccccatg gtttacggtt 720

gaagcggacg ataaacgacg tgcgcatctg aactgcatct ctcacttact cagcaaaatc 780

ccgtatgagg acattacacc tcccgccatt gatctcccac cgcgtagacc tgcgcctgaa 840

gattatgtac gtccgccgat taacgaacag ttcttcgttc ccagcatcta ttaataagaa 900

ttc 903

<210> 13
<211> 1068
<212> DNA
<213> Corynebacterium efficiens

<400> 13
catatgaata aaatggaaaa cgctccgatg ccgacgtttg gcaaagaact gcccaaactg 60

gacaacaaag cgtacaaaaa ggagctcaaa cgtctgcaag cagagctggt ggaaatgcag 120

caatgggttg tggaaacggg tactcgcgtc gtgattgtga tggaaggtcg agatgcggcc 180

ggaaaaggct ccgcgattaa gcgcatcacc cagtacctga atccgcgtac agcccgtatt 240

gaggcattac cgactcctac ctctcgcgaa aaaggtcagt ggtatttcca gaggtatgta 300

gagaagttac ctgctgcggg tgagattgtg atctttgatc gcagctggta caatcgcgcc 360

ggcgttgaac gtgttatggg cttttgcacg agtcaggaat atcgccgctt tttgcatcag 420

gcaccgatct tcgaacgctt gttagtcgaa gatggcatac atctccgcaa gtattggttc 480

tctgtatcgg atgaagaaca acttgcccgc tttcatagcc ggctgagtga tccgctgcgt 540

cgttggaaac tgtcgacgat agatctgcac agcattaccc gttgggagga ctactcacgt 600

gcgaaagacg agatgttcat tcacaccgat attcccagcg caccatggta tacagtggaa 660

tcggaggaga aaaaacgcag tcgcatcaac gtaattagcc acatcctttc aaccattccg 720

tacgagaaga ttgaccgtcc gttgccggaa atcccagaac gcccagtgcg ggaaggggag 780

tatatccgtc cacctcggaa cgaatttcgc tatgtcccgg atgttgccgc ttgtctggaa 840

gaacatcgag ttgcagcagc gcgtgaaaaa gcaaaagcag aagctaaagc aagagaagaa 900

gcggaacgtg cgctagccgc ggaaaaagtg aaagcggcca aaaaggcgaa aaagatccgt 960

aaagcccaga aggccaaagc tgcgaaaaaa gccaaaaaag ctgccggtaa ggcgaaagcg 1020

gtcaaaaaaa ccggcaaatc cgggaaaggc ggaaaataat aagaattc 1068

<210> 14
<211> 903
<212> DNA
<213> Kineococcus radiotolerans

<400> 14
catatgccgc atgttcagtt gactccggat ctgggtatga cagttcgcga tgatgaggat 60

gaacctgaac tgcttacgcc agatggcaac gtggtagata cctggcgcga agattacccg 120

tacgacgaac gtctggatcg taaggagtat gatgccgaga aacgtctgct gcaaatcgag 180

ttgctcaaac ttcagcgttg gttgaaagcc tctggtgaac gcatcgtagt gctttgcgaa 240

ggacgcgacg ctgcgggcaa aggcggcacg atcaaacgtt tcatggaaca cctgaatccg 300

cgtggtgccc gggtagttgc actggagaaa ccgtcggaac gtgaaagcac ccagtggtac 360

tttcagcgct atgtccagca cttacccgca gctggcgagt ttgtgctgtt cgatcggagc 420

tggtataatc gcgctggtgt agaacgtgtc atgggttttg cgagtcccgc agaatacgac 480

cgcttcgttg cccaagcccc gttattcgag aaaatgctcg ttgatgatgg gattcacctg 540

gtgaagttct ggttttccgt gactcgcgcg gaacaacgta cccgctttct gattcgccag 600

attgacccgg tgcgccagtg gaaactgtca ccaatggacc tggaaagcct ggaccgctgg 660

gatgaatata ccgcggcgaa agaagcgatg tttgccacca ccgatacgga tgtggcaccg 720

tggacagtcg tcaagaccaa cgacaagaaa cgcgcacgtt tagcggcaat gcgccatgtc 780

ttagcgcgct ttgactatga tgggaaagac ccagaagtgg tgggagttcc ggaccctctg 840

ctggtggttc atgctcggac gattctcgaa gcggatcgtc gccctgcctc gtaatgagaa 900

ttc 903

<210> 15
<211> 1116

<212>  DNA
<213>  Pannonibacter indicus

<400>  15
catatggacg accgtaccgt gacccgcaaa ccacgcggaa cacggccagc accagctgcg      60

gatgccgtgt tgccggtgac cgccgatagc gccgaagctg aagcgacccc tagcggcgtt     120

gctgaaacgc cggcggaggc agtgactgca ccgcaacctg ggacagaacc ggtcgccgct     180

cctgaagccg cgttagaacc ggcgattgcc cctgttgcag cagcaccccg caaaaccctg     240

gctgaaatgc gccatgatcc ggcggcgatt cacgaactgt ttgagtctgg caagtatccg     300

tatgcgaccc cgatgcgtcg cgctccgtat gaacagcgca aagcgaaact ccaagccgaa     360

ctgctgaaag cccaacggtg gatcaaagaa acgggtcaac gcgtggtcat cctgttcgag     420

ggccgtgatg cggcgggcaa aggtgggacc atcaaacgct ttatggagca tctgaatccc     480

cgtggtgcaa ctgtggttgc gttacagaaa cccacggaac aggaagccag ccagtggtac     540

tttcagcgtt acattaacca tctcccgtca ggcggtgaaa tggtcctgtt tgaccgttcg     600

tggtacaatc gggcaggagt tgagcgcgta atgggctttt gcagtgcgtc gcagtatctc     660

gagtttatgc gtcaatgtcc ggaaattgag cgcatgatgg tccgtgatgg gattcgcctg     720

tttaagtact ggttcagcgt gagtcgcgaa gaacagcgtc gccgtttcat ggagcgtcag     780

accgatccgt tgaagcagtg gaagttatca ccaatcgata ttgcctccct ggataaatgg     840

gatgattaca ccgaagcgaa agaagccatg ttcttctata cggacacagc cgacgcgccg     900

tggactatcg tgaaatccga cgataagaaa cgcgcgcgcc tgaactgcat ggagcacttc     960

cttcattctc ttccgtatcc cgacaaagac attcacctgg ttggtgtacc agatcctttg    1020

atcgtaggtg cagctcatca cgtcatttcg cacgatacgc atattctggg caaagccctg    1080

catccggaaa tgaaaccggc aggctaatga gaattc                              1116


<210>  16
<211>  813
<212>  DNA
<213>  Deinococcus radiodurans

<400>  16
catatggata tcgacaacta tcgcgtgaaa cctggtaaac gggtgaaact gtcggattgg      60

gcaacgaacg atgatgcagg cttgtccaaa gaagaaggtc aggcacaaac ggccaaactt     120

gcgggtgaat tggctgaatg gcaggaacgc ctttatgcgg aaggcaagca aagtttactg     180

ctgattctgc aagcacgcga tgcggctggg aaagatggtg ccgttaagaa ggtgattggc     240

gcgtttaacc ctgccggagt tcagatcacc agcttcaaac agccgagtgc ggaagaactg     300

tcacatgact tcttatggcg cattcatcag aaagctcccg cgaaagggta cgtaggcgta     360

tttaaccgtt ctcagtatga agatgtcttg gttactcgcg tctatgacat gatcgatgac     420

aagaccgcta aacgtcgcct ggagcatatt cggcactttg aggaactgct gacggataat 480

gccacacgta tcgtgaaagt ctacctccat attagcccgg aagagcagaa agagcgtctg 540

caagcccgtc ttgacaatcc gggaaaacac tggaaattta acccaggcga cctgaaagac 600

cgctccaatt gggacaaatt caacgatgtc tacgaggatg cgctgactac ctctaccgat 660

gatgcgccgt ggtatgtagt gccagcagat cgcaaatggt atcgtgactt agtgctctcg 720

catattctgc tgggtgccct caaggatatg aatccgcagt ttcccgcgat cgattacgac 780

ccgagcaaag ttgtgattca ctgataagaa ttc 813

<210> 17
<211> 1056
<212> DNA
<213> Gulbenkiania indica

<400> 17
atgaacgaaa aaccgctgat tccggccccg accgatgaaa ccgcggccag cagcgaacag 60

gccgcaccgg ataccccggc ggccgcggcc gcgagcgccc gtagcgcgcg cagccgtcgc 120

cgtcgcccgc cgaccgaaac cagcaaagcc catgatgaaa gcgtgcaggc gattgaagcg 180

gcccagccgg gtccggtggc cctggaagtt gcactggccc cgggcggtag caccgaagat 240

agcaccaccg ccccgctgcc ggcgtgctat ccgtatcgta cccgtatgcg tcgtccggaa 300

tatgaacgcc tgaaagcgga actgcaaatt gaactgctga aagttcagaa ctggattaaa 360

gaaaccggcc agcgcgtgat cgttctgttt gaaggtcgcg atgccgcggg taaaggcggc 420

accatcaaac gttttatgga acatctgaat ccgcgtggcg cccgtgtggt tgcgctggaa 480

aaaccgaccg aagtggaacg cggccagtgg tattttcagc gttatattca gcattttccg 540

accgccggcg aaatcgtgtt tttcgatcgt agctggtata accgcgcggg cgtggaacgt 600

gttatgggtt tttgtacccc gaatgaatat ctggaattta tgcgccaggc cccggaactg 660

gaacgtatgc tggtgaacag cggtattcgc ctgtttaaat tttggtttag cgttagccgt 720

gaagaacagc tgcgtcgctt tatcgcgcgt cgcgatgatc cgctgaaaca ttggaaactg 780

agcccgattg atatccagag cctggataaa tgggatgaat ataccgccgc gaaacagagc 840

atgtttttcc ataccgatac cgccgatgcg ccgtggaccg tgattaaaag cgatgataaa 900

aaacgtgcgc gcattaattg catccgccat tttctgcatc agctgccgta tccggataaa 960

aacccgcgtg ttgcctgtca gccggacccg ctgctggttg gcaatgcgag caaagttctg 1020

gaaccgcatg aaacccaggt tctgaccttt taataa 1056

<210> 18
<211> 948
<212> DNA
<213> Arthrobacter aurescens

<400> 18
atgaccgaag cgagcccgag cagcccgacc ggcaccagcc tggatgattg gtgggttcgt 60

gataacctgc gcgaaaccat tgatcatctg gtggaactgg gctataccat cagcggcggt 120

cagggtgaag atccggatct gattgatccg ggcggtagcg ccgttgaaac ctggaatgaa 180

gattatccgt atcagcagcg tatgacccgc gatgaatacg aaatcgaaaa ataccgcctg 240

caaatcgaac tgctgaaatt tcagtattgg ggccaggatc tgggtctgaa acatgtgatt 300

gtttttgaag gtcgtgatgc ggccggtaaa ggcggcacca tcaaacgttt taccgaacat 360

ctggacccgc gtagcgcccg taccgttgcc ctggcaaaac cgagcgatcg cgaacagggc 420

cagtggtatt ttcagcgtta tattcagcag tttccgaccg cgggtgaaat cgtgatgttt 480

gatcgtagct ggtataaccg cgccaatgtg gaacgtgtta tgggcttttg caccgatgat 540

gaatatgata cctttatggg tcaggcgccg gtgtttgaaa aaatgctggt tgatgccggt 600

atccatgtga ccaaattttg gtttagcgtt acccgccagg aacagcgtac ccgctttgcg 660

attcgtcaga tcgatccggt gcgtcgctgg aaactgagcc cgatggatct ggcgagcctg 720

gatcgctggg atgaatatac cgatgccaaa gaacgtacct ttctgcatac cgatagcgat 780

catgccccgt ggattaccat caaaagcaac gataaaaaac gtgcgcgcat taacgccatg 840

cgctattttc tgaaccagtt cgattacgaa gataaagata ccagcgtggt ttatgatgcc 900

gatccgctga ttctgcgtcg cggtcgtgat gcagtgggtg attaataa 948

<210> 19
<211> 813
<212> DNA
<213> Thiobacillus denitrificans

<400> 19
atgaaaccgc gtgatacccg cgtgaaaccg ggcagccgtg ttagcctggc ggattggcat 60

accgatggcg atgcctttgt gggtaaagat aaagcggcca gcatggcgcg tctggatgat 120

gatcgtgttc gcctggaaga actgcaagaa ctgctgtatg ccgaaggcaa acatcgcctg 180

ctggttgtgc tgcaagcgat ggataccgcc ggtaaagata gcaccattcg tcatgtgttt 240

cgcggcgttg atccgctggg tgtgcgcgtt gcgaactttg gcgtgccgag cacccatgaa 300

ctgcgtcatg attatctgtg gcgtgtgcat ccgcatgttc cggccagcgg tgaaattgcc 360

atctttaacc gtagccatta tgaagatgtg ctggttccgc gcgttaatgg cgcgattggt 420

catgccgaat gcgaacgtcg ctatcgtcag atcaatgatt ttgaacgtat gctgagcgaa 480

accggcacca ccatccgtaa attctatctg catatcagca aagatgaaca gaaaaaacgt 540

ctggaagcgc gtcgcgatac cccgaaaaaa cgctggaaat ttcagccggg tgatctggcc 600

gtgcgtgcac agtgggatga ttatcgtgcc gcctatgatg ccctgctgag cgcaaccagc 660

acccgtcatg ccccgtggca tgtggttccg gcggatgata aactggcccg caacctgatt 720

gtgagcgcgc tgctgatcga agccctggaa ggtctggata tgcgctatcc ggaaccggtg 780

gcgggcgttg cgggcacccc gattatctaa taa 813

<210> 20
<211> 1092
<212> DNA
<213> Pseudomonas fluorescens

<400> 20
atgagcgaag aaagcaccgc cctgccgctg ccgccggcac cggtgcagaa agcgccggcc 60

agcaccagcc cggcgagcac caaagcggcg ccgcgtaaag ccgcaacccc gcgtccgcgt 120

cgcccgcgta ccaccaaagc ggccccggtt aaagcggccg aagcggaaat tagcgccatc 180

agccagaaac cgatggccct gcaagtggcc aatgccccgc gtggcagcaa tgaagatagc 240

gttagcgcgg ccctgccggg taactatccg tatcgtaatc gtatgcgtcg tgcggaatat 300

gaaaaagcca aaaacgaact gcaaattgaa ctgctgaaag tgcagagctg ggttaaagaa 360

accggccagc gcatcgtggt tctgtttgaa ggtcgcgatg cggccggtaa aggcggcacc 420

attaaacgct ttatggaaca tctgaatccg cgtggcgcgc gtattgtggc cctggaaaaa 480

ccgagcgaac aggaaaaagg ccagtggtat tttcagcgtt atattcagca tctgccgacc 540

gcgggcgaaa tggtgttttt cgatcgtagc tggtataacc gcgccggtgt ggaacgtgtt 600

atggaatttt gcagcccgct gcaatatctg gaatttatgc gccagacccc ggaactggaa 660

cgtatgctgt gtaacagcgg tattctgatg ttcaaattct ggttcagcgt gaatcgcgaa 720

gaacagctgc gtcgctttat cagccgtcgc gatgatccgc tgaaacattg gaaactgagc 780

ccgattgata tcaaaagcct ggataaatgg gatgaatata ccgcggccaa acaggcgatg 840

tttttccata ccgataccgc ggatgccccg tggaccgtta tcaaaagcga tgataaaaaa 900

cgtgcgcgca ttaattgcat ccgccatttt ctgcatgaac tggattatcc gggcaaagat 960

ctgaaagtgg cccatgcgcc ggacccgctg ctggttggcc gtgccagccg cggtctggaa 1020

gaagatgaac gtacccaggc acaggccgca accgatgccg gtgcaaccaa actggccctg 1080

agcgcataat aa 1092